(19) Europäisches Patentamt
European Patent Office
Office européen des brevets

(11) Veröffentlichungsnummer: **0 028 028**
**B1**

# (12) EUROPÄISCHE PATENTSCHRIFT

(45) Veröffentlichungstag der Patentschrift: **22.05.85**

(51) Int. Cl.⁴: **C 07 D 223/22**

(21) Anmeldenummer: **80106590.5**

(22) Anmeldetag: **27.10.80**

(54) **Verfahren zur Herstellung einer Oxo-Verbindung und dazu benötigte neue Zwischenprodukte.**

(30) Priorität: **30.10.79 CH 9703/79**

(43) Veröffentlichungstag der Anmeldung: **06.05.81 Patentblatt 81/18**

(45) Bekanntmachung des Hinweises auf die Patenterteilung: **22.05.85 Patentblatt 85/21**

(84) Benannte Vertragsstaaten: **AT BE CH DE FR GB IT LI LU NL SE**

(56) Entgegenhaltungen:
**BE - A - 597 793**
**DE - A - 2 011 087**

**CHEMICAL REVIEWS, Band 36, April 1945, Baltimore, U.S.A. J.L. RIEBSOMER: "Reactions of nitrogen tetroxide with organic compounds", Seiten 157-233**
**JOURNAL OF ORGANIC CHEMISTRY, Band 28, Januar 1963, Ohio, U.S.A. W.K. SEIFERT: "Synthesis of Nitroolefins from Olefin Dinitrogen Tetroxide Adducts", Seiten 125-129**
**JOURNAL OF ORGANIC CHEMISTRY, Band 20, Oktober 1955 Ohio, U.S.A. C.R. HAUSER et al.: "Conversion of nitriles to amides and acids by means of boronfluoride", Seiten 1448-1453**

(73) Patentinhaber: **CIBA-GEIGY AG, Postfach, CH-4002 Basel (CH)**

(72) Erfinder: **Aufderhaar, Ernst, Dr., Spiegelgrund 4, CH-4303 Kaiseraugst (CH)**

(74) Vertreter: **Zumstein, Fritz, Dr. et al, Bräuhausstrasse 4, D-8000 München 2 (DE)**

**EP 0 028 028 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die Erfindung betrifft ein neues und technisch fortschrittliches Verfahren zur Herstellung des 5-Carbamoyl-10-oxo-10,11-dihydro-5H-dibenz[b,f]-azepin der Formel III, welches dadurch gekennzeichnet ist, dass man 5-Cyano-5H-dibenz[b,f]azepin der Formel I nitriert, das erhaltene 5-Cyano-10-nitro-5H-dibenz[b,f]azepin der Formel II zum 5-Carbamyl-10-nitro-5H-dibenz[b,f]azepin der Formel IV hydrolysiert, in diesem die 10-Nitrogruppe reduziert, das Reduktionsprodukt hydrolysiert und das erhaltene Endprodukt der Formel III in reiner Form isoliert.

Das erfindungsgemässe Verfahren wird durch das nachfolgende Reaktionsschema dargestellt:

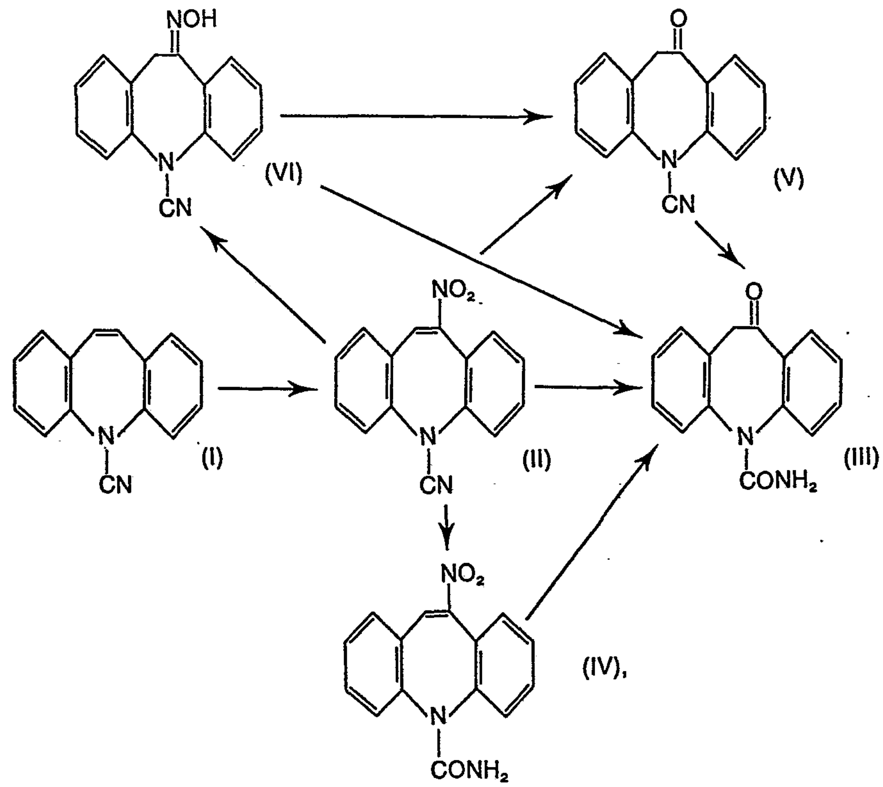

(VI) (V) (I) (II) (III) (IV),

Aus der DE-OS 2 011 087 ist ein Verfahren zur Herstellung von
5-Carbamoyl-10-oxo-10,11-dihydro-5H-dibenz-[b,f]azepin
durch Hydrolyse von 10-Methoxy-5H-dibenz[b,f]-azepin-5-carboxamid mittels wässriger Mineralsäuren bekannt. Die Zugänglichkeit dieses Ausgangsmaterials ist in der belgischen Patentschrift Nr. 597 793 angegeben, wonach z.B. 5-Acetyl-5H-dibenz[b,f]azepin zum 5-Acetyl-10,11-dihydro-10,11-dibrom-5H-dibenz[b,f]azepin bromiert, dieses in das 5-Acetyl-10-brom-5H-dibenz[b,f]azepin umgewandelt, und hieraus das 10-Methoxy-5H-dibenz[b,f]azepin hergestellt wird. Dieses wird dann durch Behandlung mit Phosgen in das entsprechende Carbonylchlorid umgewandelt aus welchem man durch Umsetzung mit Ammoniak das 10-Methoxy-5H-dibenz[b,f]azepin-5-carboxamid erhält. Gegenüber diesem, wegen seiner verhältnismässig zahlreichen Zwischenstufen umständlich durchzuführenden und überdies wegen seines hohen Verbrauchs an Brom, welches lediglich der intermediären Umwandlung von Zwischenprodukten dient, nachteiligen Herstellungsverfahren besteht das erfindungsgemässe Verfahren aus nur wenigen Verfahrensstufen, die in einfacher und übersichtlicher Weise unter Vermeidung kostspieliger Reagentien durchzuführen sind und ausserdem in hohen Ausbeuten zum Endprodukt der Formel III führen, welches in vorzüglicher Reinheit erhalten wird.

Die erfindungsgemässe Nitrierung der Verbindung I zur Verbindung der Formel II erfolgt mittels üblicher Nitrierungsmittel, z.B. Distickstofftrioxid ($N_2O_3$), gegebenenfalls im Gemisch mit Sauerstoff, z.B. Luft, oder mittels Distickstofftetroxid ($N_2O_4$) oder Gemischen solcher Verbindungen, ausserdem aber auch mittels Salpetersäure. Die Umsetzung wird in einem geeigneten Lösungsmittel vorgenommen, d.h. einem solchen, welches unter den Bedingungen der Nitrierung stabil bleibt und nicht zu unerwünschten Reaktionen mit dem Nitrierungsmittel führt. In erster Linie kommen Niederalkan- oder Halogenniederalkancarbonsäuren mit jeweils bis zu 4 Kohlenstoffatomen, z.B. Essigsäure, Propionsäure, n-Buttersäure oder Isobuttersäure, ferner z.B. Trifluor- oder Trichloressigsäure, gegebenenfalls im Gemisch mit Wasser, oder deren Anhydride, z.B. Essigsäure-, Propionsäure-, n-Buttersäure-, Isobuttersäure- oder Trifluoressigsäureanhydrid, oder Gemische solcher Carbonsäuren mit den entsprechenden Anhydriden in Betracht. Gemäss einer bevorzugten Ausführungsform des erfindungsgemässen Verfahrens verwendet man als Lösungsmittel die Anhydride der genannten Niederalkancarbonsäuren, z.B. Essigsäureanhydrid, gegebenenfalls im Gemisch mit einer Niederalkancarbonsäure, z.B. Essigsäure.

Das Verhältnis von Ausgangsmaterial zur eingesetzten Menge an Lösungsmittel (Gewicht/Volumen) kann innerhalb weiter Grenzen variieren. Vorteilhafterweise wendet man ein Verhältnis von Ausgangsmaterial zu Lösungsmittel innerhalb des Bereichs von 1 : 3 bis 1 : 30 an. Die Reaktionstemperatur liegt im Bereich von 0–120°, insbesondere von 40–80°.

Nach Chemical Reviews 36, (1945) Seite 211–212 ergibt die Umsetzung von Styrol mit Stickstofftrioxid ein Gemisch von Nitroso- und Nitroverbindungen. Ausserdem soll das Umsetzungsprodukt von Styrol mit Stickstofftrioxid zu 1-Nitro-2-phenyläthylen führen, wenn das Reaktionsprodukt der Wasserdampfdestillation unterworfen wird. Ferner wird dort angegeben, dass die Umsetzung von Stilben mit Stickstofftetroxid zum 1,2-Dinitro-1,2-diphenyläther führt. Gemäss Seite 218 der gleichen Literaturstelle liefert die Umsetzung von Cyclohexen mit trockenem Stickstofftetroxid in kaltem Petroläther das entsprechende Bis-nitroso-nitroderivat und ölige Nebenprodukte.

In J. Org. Chem. 28, (1963), Seite 125–129 wird angegeben, dass das Umsetzungsprodukt eines Olefins mit Distickstofftetroxid im wesentlichen Nitro- und Nitrosogruppen enthält, und dass dessen Umwandlung in eine Nitro-Olefinverbindung den Zusatz von Triäthylamin erfordert. So wird z.B. Cycloocten mit Distickstofftetroxid umgesetzt und das Reaktionsprodukt anschliessend mit Triäthylamin behandelt, wonach 1-Nitrocycloocten erhalten wird.

Demgegenüber wurde überraschenderweise gefunden, dass bei der erfindungsgemässen Nitrierung zusätzliche Operationen wie z.B. Wasserdampfdestillation oder Behandlung des Reaktionsgemischs mit Triäthylamin entfallen und die überaus einfach durchzuführende Aufarbeitung zu durchweg guten Ausbeuten an der Nitroverbindung der Formel II führt. Diese Verbindung ist neu und in der Literatur nicht beschrieben.

Erfindungsgemäss schliesst sich hieran die Umwandlung der Verbindung der Formel II in die Verbindung der Formel III an. Diese ist auf zwei verschiedenen Wegen möglich, welche nachfolgend als Variante (a) und Variante (b) näher beschrieben werden. In der Variante (a) wird zunächst die Cyanogruppe mittels Hydrolyse zur Carboxamidgruppe umgesetzt. Für diese Umsetzung kommen nur solche Hydrolysemethoden in Betracht, welche die an der Doppelbindung stehende 10-Nitrogruppe nicht beeinträchtigen. Zweckmässigerweise hydrolysiert man mittels saurer Mittel, z.B. Mineralsäuren, wie Schwefelsäure, Salzsäure, gegebenenfalls auch Ameisensäure. Vorzugsweise verwendet man Bortrifluorid in äquimolarer Menge oder in geringem Überschuss als Lösung in einer Niederalkan- oder Halogenniederalkancarbonsäure, z.B. solche der vorhin genannten Art, wie Essigsäure oder Trifluoressigsäure, wobei auch definierte Verbindungen von Bortrifluorid mit einer der genannten Carbonsäuren, wie Essigsäure, z.B. der Formel $BF_3 \cdot 2CH_3COOH$, verwendet werden können. Gegebenenfalls setzt man zur Durchführung der Hydrolyse dem Reaktionsgemisch ein weiteres inertes Lösungsmittel, z.B. ein solches aromatischen Charakters, wie etwa Chlorbenzol zu. Hieran schliesst sich eine Behandlung des Reaktionsgemischs bzw. der entstandenen und gegebenenfalls in reiner Form isolierten Additionsverbindung der Verbindung der Formel IV mit $BF_3$ mit hydrolysierenden Mitteln, z.B. Wasser an, wodurch das 5-Carbamoyl-10-nitro-5H-dibenz[b,f]-azepin der Formel IV in reiner Form erhalten wird.

Die Hydrolyse einer Nitrilgruppe zur Carboxamidgruppe mittels Bortrifluorid in Gegenwart von Essigsäure ist aus J. org. Chemistry 20, (1955), 1448 bekannt. Die dort angegebene Arbeitsweise erfordert hohe Temperaturen, einen grossen Überschuss an Bortrifluorid und die Verwendung wasserhaltiger Essigsäure, die zusammen mit Bortrifluorid äusserst korrosive Lösungen bildet, wodurch ihre technische Anwendung, insbesondere bei hohen Temperaturen, eingeschränkt wird und nur unter speziellen Bedingungen in technischen Apparaturen möglich ist.

Demgegenüber gestattet die beschriebene Ausführungsweise den Einsatz von Bortrifluorid in lediglich äquimolaren oder leicht überschüssigen Mengen in wasserfreien Lösungsmitteln bei Raumtemperatur. Ein weiterer Vorteil besteht darin, dass eine definierte Additionsverbindung von Bortrifluorid mit dem Hydrolyseprodukt der Formel IV in guter Reinheit und fast quantitativer Ausbeute auch aus stark verunreinigten Reaktionsgemischen isoliert und durch Behandlung mit Wasser in das reine Hydrolyseprodukt der Formel IV überführt werden kann.

Hieran schliesst sich die Umwandlung des entstandenen Zwischenprodukts der Formel IV, gegebenenfalls ohne dieses in reiner Form zu isolieren, in das Endprodukt der Formel III an. Hierzu unterwirft man die Verbindung der Formel IV reduzierenden Bedingungen, etwa der Einwirkung von katalytisch aktiviertem Wasserstoff, wie Wasserstoff in Gegenwart eines Hydrierkatalysators, etwa eines Nickel- oder Edelmetallkatalysators, z.B. Raney-Nickel oder eines Palladium-auf-Kohle Katalysators, in einem geeigneten Lösungsmittel, etwa einem Niederalkanol bis zu 4 Kohlenstoffatomen, wie Methanol oder Äthanol, oder nach nascierendem Wasserstoff, z.B. mittels eines geeigneten Metalls, wie gegebenenfalls amalgamiertem Zink, insbesondere Eisen in einer Säure, z.B. einer Mineralsäure, wie verdünnter Schwefelsäure, oder einer Carbonsäure, wie einer Niederalkancarbonsäure, z.B. einer der oben genannten, wie Essigsäure, oder eines chemischen Reduktionsmittels, z.B. Zinn(II)-chlorid·$2H_2O$, hydrolysiert das erhaltene Reduktionsprodukt im gleichen Reaktionsansatz, z.B. mittels Wasser, und isoliert das Endprodukt der Formel III in reiner

Form. Dieses wird in teilweise sehr guter Ausbeute und vorzüglicher Reinheit erhalten.

Man kann bei der Variante (a) aber auch so verfahren, dass man die Verbindung der Formel II mittels saurer Mittel, z.B. wie angegeben, wie z.B. Bortrifluorid in Essigsäure, gegebenenfalls in Gegenwart eines weiteren, inerten Lösungsmittels, etwa eines solchen aromatischen Charakters, wie Chlorbenzol, in Gegenwart von Wasser hydrolysiert, und die im Reaktionsgemisch enthaltene Verbindung der Formel IV, ohne diese zu isolieren, anschliessend, etwa wie angegeben, z.B. mittels katalytisch aktiviertem Wasserstoff, wie Wasserstoff in Gegenwart eines Hydrierkatalysators, wie eines Nickel- oder Edelmetallkatalysators, z.B. Raney-Nickel, oder eines Palladium-auf-Kohle-Katalysators, oder mittels nascierendem Wasserstoff, etwa wie angegeben, z.B. Eisen in einer Säure, etwa einer Mineralsäure, z.B. wässriger Salzsäure, oder einer Niederalkan- oder Halogenniederalkancarbonsäure, etwa wie angegeben, z.B. Essigsäure, oder dem im Reaktionsansatz bereits vorhandenen $BF_3$-Essigsäure-Wasser-Gemisch reduziert. Das im Reaktionsgemisch vorliegende Reduktionsprodukt wird, ohne isoliert zu werden, anschliessend, oder auch gleichzeitig, mittels eines sauren Mittels etwa wie angegeben, z.B. mittels einer wässrigen Säure, etwa dem im Reaktionsgemisch vorliegenden $BF_3$-Essigsäure-Wasser-Gemisch, hydrolysiert, und das entstandene Endprodukt der Formel III in reiner Form isoliert.

Die Verbindung der Formel IV und deren Addukt mit Bortrifluorid ist neu und in der Literatur nicht beschrieben.

In der Variante (b) geht man so vor, dass man zuerst in der Verbindung der Formel II die 10-Nitrogruppe reduziert und dann das Reduktionsprodukt entweder direkt oder über das
5-Cyano-10-oxo-10,11-dihydro-5H-dibenz-[b,f]azepin
der Formel V zum Endprodukt der Formel III hydrolysiert. Hierbei kann im 5-Cyano-10-nitro-5H-dibenz[b,f]azepin der Formel II die Nitrogruppe nach einer der oben beschriebenen Methoden reduziert und das im Reaktionsgemisch entstandene Zwischenprodukt mittels Hydrolyse in die Verbindung der Formel V überführt werden. Die Reduktion kann, wie vorhin angegeben, z.B. mittels katalytisch aktiviertem Wasserstoff, wie Wasserstoff in Gegenwart eines Hydrierkatalysators, etwa eines Nickel- oder Edelmetallkatalysators, z.B. Raney-Nickel oder eines Palladium-auf-Kohle Katalysators, in einem geeigneten Lösungsmittel, z.B. einen Niederalkanol mit bis zu 4 Kohlenstoffatomen, z.B. Methanol oder Äthanol, oder mittels nascierendem Wasserstoff, etwa mittels eines geeigneten Metalls, wie gegebenenfalls amalgamiertem Zink oder insbesondere Eisen in einer Säure, z.B. einer Mineralsäure, wie verdünnter Schwefelsäure oder konz. Salzsäure, oder einer Niederalkancarbonsäure, etwa einer der oben genannten, z.B. Essigsäure, oder mittels eines chemischen Reduktionsmittels, wie etwa Zinn(II)-chlorid·$2H_2O$ durchgeführt werden. Hierbei können zusätzliche Lösungsmittel, etwa ein Niederalkanol mit 1 bis 4 Kohlenstoffatomen, z.B. Äthanol, oder ein Niederalkoxyniederalkanol mit jeweils bis zu 4 Kohlenstoffatomen im Niederalkoxy- und im Niederalkanolteil, z.B. 2-Methoxy- oder 2-Äthoxyäthanol und/oder ein Lösungsmittel aromatischen Charakters, z.B. ein gegebenenfalls niederalkyliertes, wie methyliertes, oder ein halogeniertes, wie chloriertes Benzol, wie Benzol, Toluol, oder Chlorbenzol eingesetzt werden. Die Reaktionstemperatur liegt in einem Bereich von 10 bis 100°, vorzugsweise von 30–70°. Das Reaktionsgemisch lässt sich, zweckmässigerweise nach Entfernen unlöslicher Anteile, der Hydrolyse, z.B. der Einwirkung von Wasser, unterwerfen, und nach Aufarbeiten kann das 5-Cyano-10-oxo-10,11-dihydro-5H-dibenz[b,f]azepin der Formel V in reiner Form isoliert werden, welches in guten Ausbeuten und vorzüglicher Reinheit erhalten wird. Diese Verbindung ist neu und in der Literatur nicht beschrieben. Eine bevorzugte Ausführungsform der beschriebenen Umwandlung der Verbindung der Formel II in die der Formel V besteht darin, dass man für die Reduktion mittels eines Metalls, z.B. Eisen, wie oben beschrieben, ein Lösungsmittel einsetzt, welches entstehende Metall-, z.B. Eisensalze, in Lösung hält und so das Auftreten eines schwer filtrierbaren Niederschlags, etwa in Form eines Schlamms, weitgehend verhindert. Solche Lösungsmittel sind z.B. stark polare organische Lösungsmittel, etwa Niederalkyläther des Äthylenglycols, worin Niederalkyl bis zu 4 Kohlenstoffatome hat und z.B. Methyl oder Äthyl darstellt, und demnach z.B. Äthylenglycol-monoäthyläther ist.

Hieran schliesst sich die Umwandlung der Cyanogruppe in der Verbindung der Formel V in die Carboxamidgruppe des Endproduktes der Formel III mittels Hydrolyse an. Diese kann mittels basischer oder saurer Mittel durchgeführt werden. Als basische Mittel kommen hierzu etwa die Oxide oder Hydroxide von Erdalkali- oder Alkalimetallen, z.B. Magnesium- oder Calciumhydroxid, ferner z.B. Natriumhydroxid, gegebenenfalls in Gegenwart eines Peroxids, wie Wasserstoffperoxid, oder ein Alkalimetallbicarbonat, wie Natriumbicarbonat, im Gemisch mit Wasserstoffperoxid in Betracht, als saure Mittel z.B. Mineralsäuren, wie Schwefelsäure oder Polyphosphorsäure, ferner Niederalkan- oder Halogenniederalkancarbonsäuren mit bis zu 4 Kohlenstoffatomen, z.B. Ameisen- oder Essigsäure bzw. Trichlor- oder Trifluoressigsäure im Gemisch mit Mineralsäuren, z.B. konz. Schwefelsäure. Saure Mittel sind ferner z.B. Lewis-Säuren, z.B. Bortrifluorid, welches als Lösung in einer Niederalkancarbonsäure der oben beschriebenen Art, wie Essigsäure, aber auch als definierte Verbindung, z.B. der Formel $BF_3 \cdot 2\,CH_3COOH$ vorliegen kann. Gegebenenfalls fügt man dem Reaktionsgemisch noch ein weiteres Lösungsmittel, z.B. ein solches aromatischen Charakters, wie etwa Chlorbenzol zu. Die Reaktionstemperaturen liegen im Bereich von −5 bis +150°, vorzugsweise von 0–40°.

Eine weitere Ausführungsweise der Variante (b) besteht darin, dass man zur Herstellung des Zwischenprodukts der Formel V die 10-Nitrogruppe in der Verbindung der Formel II reduziert, das erhaltene

5-Cyano-10-isonitroso-10,11-dihydro-5H-dibenz[b,f]azepin

der Formel VI in reiner Form isoliert, dieses zum 5-Cyano-10-oxo-10,11-dihydro-5H-dibenz[b,f]azepin der Formel V hydrolysiert, und dieses in reiner Form isoliert. Bei dieser Ausführungsweise wird die Nitrogruppe in der Verbindung der Formel II zu der der Formel VI entsprechenden 10-Isonitrosoverbindung, etwa wie angegeben, z.B. mittels Zinkstaub in einer Säure, etwa einer Niederalkancarbonsäure der angegebenen Art, wie Essigsäure, gegebenenfalls in Gegenwart eines inerten Lösungsmittels, wie eines Niederalkanols der angegebenen Art, z.B. Äthanol, oder mittels Wasserstoff in Gegenwart eines Hydrierkatalysators, wie eines Edelmetallkatalysators z.B. eines Palladium-auf-Kohle Katalysators in einem geeigneten Lösungsmittel, etwa eines solchen aromatischen Charakters, z.B. Pyridin, reduziert, und das erhaltene

5-Cyano-10-isonitroso-10,11-dihydro-5H-dibenz[b,f]azepin

der Formel VI in reiner Form isoliert. Dieses wird in guter Ausbeute und vorzüglicher Reinheit erhalten. Die Verbindung ist neu und in der Literatur nicht beschrieben. Die Verbindung der Formel VI wird anschliessend mittels Hydrolyse in die Verbindung der Formel V umgewandelt, indem man, insbesondere mittels saurer Mittel, etwa wie angegeben, wie einer Säure, etwa einer Mineralsäure z.B. Salzsäure oder Schwefelsäure, oder einer Niederalkan- oder Halogenniederalkancarbonsäure, z.B. Essigsäure oder Trifluoressigsäure in Gegenwart von Wasser hydrolysiert. Gegebenenfalls fügt man dem Reaktionsgemisch noch ein zusätzliches Lösungsmittel, z.B. ein solches aromatischen Charakters, wie z.B. ein gegebenenfalls durch Niederalkyl mit bis zu 4 Kohlenstoffatomen, wie etwa Methyl, oder durch Halogen, wie Chlor, substituiertes Benzol, wie Benzol, Toluol oder Chlorbenzol, oder eine Niederalkancarbonsäure, etwa der genannten Art, wie z.B. Essigsäure, oder ein Niederalkanol mit bis zu 4 Kohlenstoffatomen, z.B. Äthanol zu, oder man verwendet Gemische solcher Lösungsmittel. Aus dem Reaktionsgemisch wird die Verbindung der Formel V in reiner Form isoliert, welche dann, wie oben angegeben, in das Endprodukt der Formel III umgewandelt wird.

Eine weitere Ausführungsweise von Variante (b) zur Herstellung des Endproduktes der Formel III besteht darin, dass man die Verbindung der Formel VI hydrolysiert und das erhaltene Endprodukt der Formel III in reiner Form isoliert.

Gemäss dieser Ausführungsweise wird in der Verbindung der Formel VI sowohl die 5-Cyanogruppe als auch die 10-Isonitrosogruppe im gleichen Reaktionsansatz hydrolysiert. So kann man z.B. in der Verbindung der Formel VI die 10-Isonitrosogruppe mittels saurer Mittel, etwa wie angegeben, z.B. mittels einer Mineralsäure, wie Salzsäure in die 10-Oxogruppe umwandeln, und anschliessend das Reaktionsgemisch, z.B. wie beschrieben, mit einer Säure, z.B. einer Mineralsäure, wie Schwefelsäure oder Polyphosphorsäure, oder mit einer Carbonsäure, z.B. einer Niederalkan- oder Halogenniederalkancarbonsäure, wie Essigsäure oder Trifluoressigsäure, oder einer Lewis-Säure, wie Bortrifluorid in Gegenwart einer Carbonsäure, z.B. Essigsäure oder Gemischen solcher Säuren, gegebenenfalls in Gegenwart eines weiteren inerten Lösungsmittels, wie eines Niederalkanols mit bis zu 5 Kohlenstoffatomen, wie Methanol oder Äthanol, in Gegenwart von Wasser hydrolysieren und das Endprodukt der Formel III in reiner Form isolieren.

Die neuen Zwischenprodukte der Formeln II, IV, (letzteres auch in Form des $BF_3$-Addukts) V und VI bilden ebenfalls Gegenstände der Erfindung. Die folgenden Beispiele dienen zur Illustration der Erfindung. Sie betreffen jeweils einzelne Schritte des erfindungsgemässen Gesamtverfahrens und sind zu diesem in der vorstehend angegebenen Weise zu kombinieren. Temperaturen sind in Celsiusgraden angegeben.

Beispiel 1

6,0 g (0,027 Mol) 5-Cyano-5H-dibenz[b,f]azepin werden in der Mischung von 80 ml Acetanhydrid und 20 ml Essigsäure gelöst. Man heizt auf 50° und fügt tropfenweise im Laufe von 1½ Stunden die Lösung von 5,6 g (0,08 Mol) Natriumnitrit in 10 ml Wasser zu, wobei man die Temperatur nicht über 55° steigen lässt.

Man hält noch 2 Stunden bei 50° und destilliert dann das Lösungsmittel bei reduziertem Druck und einer Badtemperatur von 50° ab. Der Rückstand wird zweimal mit jeweils 100 ml Eiswasser digeriert und in 80 ml Äthanol aufgenommen.

Nach mehrstündigem Stehen bei 0° werden die ausgefallenen gelben Kristalle abgesaugt und mit wenig Äthanol gewaschen. Das erhaltene 5-Cyano-10-nitro-5H-dibenz[b,f]azepin schmilzt bei 175–176° unter Zersetzung.

Ausbeute: 5,2 g, 72% d. Th.

Die analytischen und spektroskopischen Daten sind im Einklag mit der angenommenen Struktur.

Beispiel 2

6,5 g (0,03 Mol) 5-Cyano-5H-dibenz[b,f]azepin werden in der Mischung von 100 ml Essigsäure und 100 ml Acetanhydrid gelöst. Man heizt auf 40° und fügt im Laufe von 45 Min. 6,2 g (0,09 Mol) Natriumnitrit zu, wobei man einen langsamen Luftstrom durch die Lösung leitet. Die Temperatur steigt ohne weitere Heizung auf 55° und wird nach dem Ende der Nitritzugabe noch eine Stunde auf 55° gehalten.

Man destilliert das Lösungsmittel im Vakuum bei einer Badtemperatur von 50° ab, nimmt den Rückstand in 300 ml Toluol auf, schüttelt zur Entfernung der anorganischen Bestandteile mehrmals mit Wasser aus und destilliert das Toluol im Vakuum bis auf 40 ml ab. Das ausgefallene gelbe Produkt wird abgesaugt und mit wenig Toluol gewaschen; es ist identisch mit 5-Cyano-10-nitro-5H-dibenz[b,f]azepin gemäss Beispiel 1.

Ausbeute: 6,1 g; 77,5% d. Th.

Beispiel 3

19,6 g (0,09 Mol) 5-Cyano-5H-dibenz[b,f]azepin werden wie in Beispiel 2 in 300 ml Essigsäure und 300 ml Acetanhydrid mit 18,6 g (0,27 Mol) Natriumnitrit umgesetzt. Der nach dem Abdampfen des Lösungsmittels zurückbleibende rotgelbe Sirup wird mit 300 ml Wasser bis zur vollständigen Verfestigung durchgerührt. Man saugt ab, wäscht mit Wasser bis zur neutralen Reaktion des Filtrats und trocknet im Vakuum.

Ein gleiches Rohprodukt wird erhalten, wenn die obige Umsetzung bei einer Temperatur von 80–85° durchgeführt wird.

Das erhaltene Rohprodukt ist für weitere Umsetzungen geeignet, kann aber auch wie folgt gereinigt werden:

1. 23,7 g Rohprodukt werden aus Isopropanol umkristallisiert und liefern ein gelbes kristallines Material, welches identisch ist mit 5-Cyano-10-nitro-5H-dibenz[b,f]azepin gemäss Beispiel 1. Ausbeute: 18,0 g, 76,2% d. Th.
2. Ein bezüglich Ausbeute und Qualität gleiches Endprodukt wird durch Digerieren des Rohprodukts mit Essigsäure erhalten.

Beispiel 4

In einem Kolben erzeugt man durch langsames Zutropfen von 20%iger Schwefelsäure zu einer konzentrierten wässrigen Lösung von 40,0 g (0,58 Mol) Natriumnitrit nitrose Gase ($N_2O_3$), welche durch einen langsamen Luftstrom in die auf 55° erhitzte Lösung von 10,9 g (0,05 Mol) 5-Cyano-5H-dibenz[b,f]azepin in 100 ml Toluol eingeleitet werden. Der zugeführte Luftsauerstoff wird dabei so gering gehalten, dass sich keine explosionsfähigen Toluoldampf-Sauerstoff-Gemische bilden können. Man setzt das Einleiten bis zur vollständigen Umsetzung des Ausgangsmaterials fort (DC-Kontrolle), vertreibt den $N_2O_3$-Überschuss durch einen lebhaften Stickstoffstrom und dampft das Toluol unter vermindertem Druck bei einer Temperatur von 40° ab. Der zurückbleibende rote Sirup wird in 100 ml Isopropanol aufgenommen. Nach mehrstündigem Stehen bei 5° wird das Kristallisat abgesaugt und mit wenig Isopropanol gewaschen. Das erhaltene 5-Cyano-10-nitro-5H-dibenz[b,f]azepin ist identisch mit dem Produkt gemäss Beispiel 1.

Ausbeute: 9,4 g, 71,7% d. Th.

Beispiel 5

Analog der im Beispiel 4 beschriebenen Arbeitsweise erzeugt man in einem Kolben aus 55,0 g (0,8 Mol) Natriumnitrit nitrose Gase und treibt diese mittels eines langsamen Luftstroms in die auf 50° gehaltene Lösung von 10,9 g (0,05 Mol) 5-Cyano-5H-dibenz[b,f]azepin in 110 ml Essigsäure und 100 ml Acetanhydrid. Nach 3 Stunden ist die Umsetzung beendet; anschliessend wird das Lösungsmittel unter Vakuum bei einer Badtemperatur von 50° abdestilliert, der Rückstand in 50 ml Isopropanol aufgenommen und das auskristallisierte Material nach mehrstündigem Stehen bei 20° abgesaugt. Man erhält 5-Cyano-10-nitro-5H-dibenz[b,f]azepin, welches mit dem gemäss Beispiel 1 erhaltenen Produkt identisch ist.

Ausbeute: 9,5 g, 72,5% d. Th.

Beispiel 6

Analog der im Beispiel 4 beschriebenen Arbeitsweise erzeugt man in einem Kolben aus 30,0 g (0,43 Mol) Natriumnitrit nitrose Gase und treibt diese mittels eines langsamen Luftstroms in die auf 55° gehaltene Lösung von 10,9 g (0,05 Mol) 5-Cyano-5H-dibenz[b,f]azepin in 110 ml Acetanhydrid. Nach beendeter Umsetzung (DC-Kontrolle) wird das Reaktionsgemisch unter Vakuum bei einer Badtemperatur von 50° eingedampft und der Rückstand mit 20 ml Essigsäure aufgenommen. Man lässt 2 Stunden bei 20° stehen, saugt das Kristallisat ab und wäscht mit wenig Essigsäure. Man erhält das 5-Cyano-10-nitro-5H-dibenz[b,f]-azepin, welches mit dem gemäss Beispiel 1 erhaltenen Produkt identisch ist.

Ausbeute: 9,1 g, 69,4% d. Th.

Beispiel 7

10,0 g (0,046 Mol) 5-Cyano-5H-dibenz[b,f]azepin werden in 100 ml Toluol bei 55° gelöst. Man leitet in die Lösung aus einer Druckflasche unter Rühren 5,0 g (0,054 Mol) $N_2O_4$ langsam ein, wobei die Temperatur bis auf 60° steigt, hält anschliessend das Reaktionsgemisch auf dieser Temperatur, bis alles Ausgangsmaterial umgesetzt ist (DC-Kontrolle), kühlt dann auf 20° und trocknet die Toluolphase über Natriumsulfat. Nach dem Eindampfen im Vakuum erhält man ein rotes Öl, das in 50 ml Isopropanol aufgenommen wird. Nach mehrstündigem Stehen bei 20° werden die Kristalle abgesaugt und mit wenig Isopropanol gewaschen. Das erhaltene 5-Cyano-10-nitro-5H-dibenz[b,f]azepin ist identisch mit dem gemäss Beispiel 1 erhaltenen Produkt.

Ausbeute: 6,7 g, 56% d. Th.

Beispiel 8

Man löst 43,6 g (0,2 Mol) 5-Cyano-5H-dibenz[b,f]azepin in 250 ml Essigsäure bei 55° und leitet in die Lösung aus einer Druckflasche unter Rühren während 2½ Stunden $N_2O_4$ ein, wobei durch gelegentliche Kühlung die Temperatur auf 55° gehalten wird. Das Ende der Reaktion wird an einer Grünfärbung der Lösung ($N_2O_4$-Überschuss) sowie durch DC-Kontrolle erkannt. Man lässt abkühlen, rührt mehrere Stunden bei Raumtemperatur nach, und filtriert dann den ausgefallenen Niederschlag ab, der mit wenig Essigsäure gewaschen wird. Durch Einengen des Filtrates und Aufnehmen mit Acetonitril erhält man ein zweites Kristallisat von 5-Cyano-10-nitro-5H-dibenz[b,f]azepin, welches mit dem gemäss Beispiel 1 erhaltenen Produkt identisch ist.

Totalausbeute: 19,5 g, 37% d. Th.

Beispiel 9

43,6 g (0,2 Mol) 5-Cyano-5H-dibenz[b,f]azepin werden in 250 ml Essigsäure bei 55° gelöst. Hierzu

tropft man bis zur beginnenden Trübung 60 ml Wasser, und leitet dann aus einer Druckflasche langsam $N_2O_4$ ein, bis im DC kein Ausgangsmaterial mehr nachweisbar ist. Man kühlt auf 5° ab und rührt 2 Stunden bei dieser Temperatur, filtriert dann das Kristallisat ab und wäscht mit 80%iger Essigsäure. Man erhält 5-Cyano-10-nitro-5H-dibenz[b,f]azepin, welches mit dem gemäss Beispiel 1 erhaltenen Produkt identisch ist.

Ausbeute: 42,1 g, 80% d. Th.

Beispiel 10

43,6 g (0,2 Mol) 5-Cyano-5H-dibenz[b,f]azepin werden in 175 ml Essigsäure bei 55° gelöst. In die Lösung leitet man bei dieser Temperatur $N_2O_4$ aus einer Druckflasche so lange ein, bis alles Ausgangsmaterial umgesetzt ist (DC-Kontrolle) und das Produkt ausfällt. Man gibt dann portionenweise unter gelegentlicher Kühlung 16,4 g (0,2 Mol) Natriumacetat zu und hält die Temperatur auf 50–55°. Anschliessend wird 3 Std. bei Raumtemperatur gerührt, filtriert und das Kristallisat mit Essigsäure und Wasser nachgewaschen. Man erhält 5-Cyano-10-nitro-5H-dibenz[b,f]azepin, welches mit dem gemäss Beispiel 1 erhaltenen Produkt identisch ist.

Ausbeute: 36,1 g, 68,6% d. Th.

Beispiel 11

21,8 g (0,1 Mol) 5-Cyano-5H-dibenz[b,f]azepin werden in 110 ml Acetanhydrid bei 55° gelöst. In diese Lösung leitet man aus einer Druckflasche 10,0 g (0,1 Mol) $N_2O_4$ so langsam ein, dass keine kunkelbraunen Gase entweichen, und hält durch gelegentliche Kühlung die Temperatur bei 55°. Nach dem Ende der Reaktion wird während einer Stunde ein kräftiger Stickstoffstrom durchgeleitet, dann auf −20° abgekühlt und 2 Stunden auf dieser Temperatur gehalten. Danach saugt man das gelbe Kristallisat ab und wäscht mit wenig Acetonitril nach, wonach man das 5-Cyano-10-nitro-5H-dibenz[b,f]azepin, welches mit dem gemäss Beispiel 1 erhaltenen Produkt identisch ist, erhält. Das Filtrat wird im Vakuum bei einer Badtemperatur von 50° zu einem roten Öl eingedampft. Man nimmt mit 10 ml Acetonitril auf, lässt 2 Stunden bei 5° stehen und saugt die zweite Kristallisation ab.

Totalausbeute: 19,5 g, 74,1% d. Th.

Beispiel 12

21,8 g (0,1 Mol) 5-Cyano-5H-dibenz[b,f]azepin werden in 140 ml Acetanhydrid bei 50° gelöst. In diese Lösung leitet man aus einer Druckflasche unter Rühren langsam 12,0 g (0,13 Mol) $N_2O_4$ ein, wobei man die Temperatur durch Kühlung zwischen 50 und 55° hält. Man lässt eine Stunde nachreagieren, leitet dann einen kräftigen Stickstoffstrom durch und setzt langsam 60 ml Wasser zu, wobei durch Kühlung die Temperatur bei 50 bis 55° gehalten wird. Anschliessend wird auf 5° gekühlt, eine Stunde kristallisieren gelassen und abfiltriert. Man erhält 5-Cyano-10-nitro-5H-dibenz[b,f]azepin, welches mit dem gemäss Beispiel 1 erhaltenen Produkt identisch ist. Das Filtrat wird unter Vakuum eingedampft und liefert nach Aufnehmen des Rückstands in 40 ml 80%iger Essigsäure ein zweites Kristallisat.

Totalausbeute 21,5 g, 81,7% d. Th.

Beispiel 13

43,6 g (0,2 Mol) 5-Cyano-5H-dibenz[b,f]azepin werden in 430 ml Acetanhydrid gelöst. In diese Lösung leitet man unter Rühren und gelegentlicher Kühlung aus einer Druckflasche 19,0 g (0,206 Mol) $N_2O_4$ so langsam ein, dass die Temperatur nicht über 25° steigt. Nach dem Ende der Umsetzung verfärbt sich die Lösung grün, und das Produkt fällt kristallin aus. Man rührt eine Stunde unter Eiskühlung und Durchleiten eines kräftigen Stickstoffstroms, filtriert dann ab und wäscht mit wenig Essigsäureäthylester. Man erhält 5-Cyano-10-nitro-5H-dibenz[b,f]azepin welches mit dem gemäss Beispiel 1 erhaltenen Produkt identisch ist. Aus dem Filtrat erhält man nach Eindampfen im Vakuum und Aufnehmen mit Essigsäureäthylester ein zweites Kristallisat.

Totalausbeute: 42,3 g, 80,4% d. Th.

Beispiel 14

35,0 g (0,16 Mol) 5-Cyano-5H-dibenz[b,f]azepin werden in 160 ml Acetanhydrid bei 20° suspendiert. Hierzu lässt man unter Rühren die Lösung von 14,7 g (0,16 Mol) $N_2O_4$ in 160 ml Acetanhydrid langsam im Laufe von 5 Stunden tropfen, wobei die Temperatur zwischen 20 und 25° gehalten wird. Nach vollständiger Umsetzung des Ausgangsmaterials (DC-Kontrolle) kühlt man unter Durchleiten eines kräftigen Stickstoffstroms für eine Stunde auf 0–5° ab und saugt das kristalline Produkt ab. Man erhält 5-Cyano-10-nitro-5H-dibenz[b,f]azepin, das mit dem gemäss Beispiel 1 erhaltenen Produkt identisch ist.

Aus dem Filtrat erhält man nach Eindampfen im Vakuum auf 50 ml ein zweites Kristallisat.

Totalausbeute: 34,6 g, 80% d. Th.

Beispiel 15

43,6 g (0,2 Mol) 5-Cyano-5H-dibenz[b,f]azepin werden in 175 ml Essigsäureanhydrid bei 50° gelöst. Während zwei Stunden leitet man in diese Lösung aus einer Druckflasche soviel $N_2O_4$ ein, dass das Ausgangsmaterial vollständig umgesetzt wird und ein leichter Überschuss $N_2O_4$ im Abgas erkennbar wird. Man lässt das Produkt bei 50° auskristallisieren und setzt dann portionsweise 16,5 g (0,2 Mol) Natriumacetat zu. Nach Abklingen der Wärmeentwicklung rührt man noch 30 Min. bei 50°, danach mehrere Stunden bei Raumtemperatur nach. Nach Filtration und Waschen des Kristallisats mit Essigsäure und Wasser erhält man 5-Cyano-10-nitro-5H-dibenz[b,f]azepin, welches mit dem gemäss Beispiel 1 erhaltenen Produkt identisch ist. Durch Aufarbeiten der Mutterlauge können weitere 3 g der Verbindung erhalten werden.

Totalausbeute: 44,1 g, 83,8% d. Th.

Beispiel 16

Zu einer Lösung von 2,0 g 5-Cyano-5H-dibenz[b,f]azepin in 10 ml Essigsäureanhydrid tropft man bei 20° unter Rühren 5 ml konz. Salpetersäure (ca. 64%ig) zu, wobei eine exotherme Reaktion unter Farbveränderung nach Tiefgelb abläuft. Man lässt eine Stunde bei 20° nachreagieren, tropft dann bei 50° 40 ml Wasser zu und nimmt die ausgefällten Schmieren in Essigsäureäthylester auf. Der nach Waschen und Eindampfen der organischen Phase hinterbleibende Rückstand liefert mit Acetonitril bei längerem Stehen 0,6 g gelbe Kristalle, die nach liquidchromatographischer Kontrolle 78% 5-Cyano-10-nitro-5H-dibenz[b,f]azepin enthalten.

Beispiel 17

Eine Suspension von 26,3 g (0,1 Mol) 5-Cyano-10-nitro-5H-dibenz[b,f]azepin in 100 ml Essigsäure wird bei Raumtemperatur mit 50 ml einer Lösung von 15 Gew.-% $FB_3$ in Essigsäure (= 0,11 Mol) versetzt. Hierbei steigt die Temperatur langsam auf 34° unter vollständiger Auflösung des Ausgangsmaterials. Man setzt bei 30° im Laufe von 5 Min. 30 ml Wasser zu, was zu einem erneuten Temperaturanstieg auf 37° führt. Bei dieser Temperatur werden im Laufe von 20 Min. portionenweise 40 g Eisenpulver eingetragen, wobei man die Temperatur durch gelegentliche Kühlung auf 65–70° hält. Nach dem Abklingen der exothermen Reaktion wird noch 15 Min. gerührt und anschliessend von anorganischem Material abfiltriert, das dreimal mit wenig Essigsäure nachgewaschen wird. Das gesamte Filtrat wird unter gutem Rühren zu 1½ l Wasser getropft, die entstandene Fällung nach 2stündigem Rühren abfiltriert und mit Wasser neutral gewaschen. Nach Trocknen bei 60° im Vakuum erhält man

5-Carbamoyl-10-oxo-10,11-dihydro-5H-dibenz-[b,f]azepin,

welches aufgrund des IR-Spektrums mit authentischem Material identisch ist.

Ausbeute: 23,0 g, 91,2% d. Th.

Beispiel 18

Zu einer Suspension von 26,3 g (0,1 Mol) 5-Cyano-10-nitro-5H-dibenz[b,f]azepin in der Mischung von 260 ml Chlorbenzol und 130 ml Essigsäure fügt man rasch 50 ml einer Lösung von 15 Gew.-% $BF_3$ in Essigsäure zu und erhält unter schwacher Erwärmung eine klare gelbe Lösung. Man rührt das Reaktionsgemisch während 10 Min. und setzt dann 40 g Eisenpulver in einer Portion zu. Unter gutem Rühren werden 100 ml Wasser im Laufe von 30 Min. zugetropft, wobei die Temperatur auf 65° steigt. Man hält durch äussere Heizung die Temperatur während 2 Stunden auf 60–65°, filtriert vom anorganischen Teil ab und wäscht mit Chlorbenzol und Essigsäure nach. Nach Abtrennen der Chlorbenzolphase wird diese mit Wasser bis zur beginnenden Kristallisation des Produkts gewaschen. Man engt im Vakuum ein und nimmt den Rückstand mit 100 ml Methanol auf. Nach Absaugen und Waschen mit wenig Methanol erhält man 5-Carbamoyl-10-oxo-10,11-dihydro-5H-dibenz[b,f]azepin, das aufgrund des IR-Spektrums mit authentischem Material identisch ist.

Ausbeute: 21,4 g 85% d. Th.

Beispiel 19

Zu einer Suspension von 26,3 g (0,1 Mol) 5-Cyano-10-nitro-5H-dibenz[b,f]azepin in 200 ml Essigsäure lässt man rasch 50 ml einer Lösung von 15 Gew.-% $BF_3$ in Essigsäure zufliessen. Man rührt 45 Min. bis zur vollständigen Auflösung und zum Abklingen der mässig exothermen Reaktion. Bei 30° werden dann 40 g Eisenpulver zugesetzt und 100 ml Wasser langsam im Laufe von 30 Min. zugetropft, wobei die Temperatur auf 65° ansteigt. Man rührt 15 Std. bei Raumtemperatur, erwärmt nochmals auf 60°, filtriert vom Ungelösten ab und wäscht mit Essigsäure dreimal nach. Das Filtrat wird im Vakuum auf ein Volumen von ca. 100 ml eingedampft und tropfenweise mit 400 ml Wasser versetzt. Man rührt das Gemisch noch während 2 Stunden, filtriert ab und wäscht den Filterkuchen mit Wasser neutral. Nach Trocknen bei 50° im Vakuum erhält man 23,8 g (94,4% d. Th.) Rohprodukt, das aus 200 ml Essigsäure/Wasser (8 : 2) umkristallisiert wird. Man erhält reines

5-Carbamoyl-10-oxo-10,11-dihydro-5H-dibenz-[b,f]azepin,

welches aufgrund des IR-Spektrums identisch mit authentischem Material ist.

Ausbeute: 19,7 g, 78% d. Th.

Beispiel 20

31,5 g (0,12 Mol) 5-Cyano-10-nitro-5H-dibenz-[b,f]azepin werden in 340 ml Chlorbenzol suspendiert und rasch mit 98 ml einer Lösung von 10 Gew.-% $BF_3$ in Essigsäure versetzt. Unter Erwärmung auf 30° tritt Auflösung ein, kurz darauf beginnt das $BF_3$-Addukt des 5-Carbamoyl-10-nitro-5H-dibenz[b,f]azepins auszufallen. Man lässt im Eisbad 2 Std. kristallisieren, saugt ab und wäscht mit Benzin nach. Das trockene Zwischenprodukt wird in der Mischung von 200 ml Essigsäure und 35 ml Wasser gelöst. Man setzt im Laufe von 30 Min. portionenweise 50 g Eisenpulver zu und hält durch Kühlung die Temperatur auf ca. 60°. Man rührt noch eine Stunde bei 50°, filtriert und wäscht mit Essigsäure nach. Das Filtrat wird im Vakuum eingedampft und der Rückstand mit 500 ml Wasser aufgenommen. Das ausgefallene Produkt wird abgesaugt, mit Wasser neutral gewaschen und im Vakuum bei 50° getrocknet. Man erhält 5-Carbamoyl-10-oxo-10,11-dihydro-5H-dibenz[b,f]azepin, welches aufgrund des IR-Spektrums mit authentischem Material identisch ist.

Ausbeute: 26,0 g, 86% d. Th.

Beispiel 21

Zu einer Suspension von 4,0 g (0,015 Mol) 5-Cyano-10-nitro-5H-dibenz[b,f]azepin in 40 ml Essigsäure fügt man tropfenweise 2 ml konz. Schwefelsäure zu und rührt weitere 15 Stunden bei Raumtemperatur. Die klare Lösung wird nach und nach mit 100 ml Wasser versetzt, das ausgefällte Material in Chloroform aufgenommen, die Chloroformschicht mit Wasser gewaschen und zur Trockne verdampft. Nach dem Umkristallisieren des Rückstandes aus Isopropanol erhält man reines 5-Carbamoyl-10-nitro-5H-dibenz[b,f]azepin; Smp. 208–212°, Ausbeute 2,2 g, 52,4% d. Th. Die analytischen und spektroskopischen Daten stehen mit der angegebenen Struktur in Einklang.

Beispiel 22

10 g (0,038 Mol) 5-Cyano-10-nitro-5H-dibenz[b,f]azepin werden in 100 ml 98%iger Ameisensäure 5 Std. auf 90–100° erhitzt. Man lässt auf Raumtemperatur abkühlen und setzt dann bis zur beginnenden Trübung 90 ml Wasser zu, lässt 15 Stunden kristallisieren, filtriert, wäscht die Kristalle mit Wasser und trocknet bei 40° im Vakuum. Man erhält 5-Carbamoyl-10-nitro-5H-dibenz[b,f]-azepin, welches identisch mit dem gemäss Beispiel 21 erhaltenen Produkt ist.

Ausbeute: 9,1 g, 85% d. Th.

Beispiel 23

Zu einer Suspension von 13,1 g (0,05 Mol) 5-Cyano-10-nitro-5H-dibenz[b,f]azepin in 130 ml Chlorbenzol lässt man unter Rühren rasch 25 ml einer Lösung von 15 Gew.-% $BF_3$ in Essigsäure zulaufen und erhält eine klare bräunliche Lösung, deren Temperatur auf 35° steigt. Die nach einigen Minuten beginnende Kristallisation wird durch einstündiges Rühren im Eisbad vervollständigt. Man saugt ab, wäscht mit Chlorbenzol und trocknet im Vakuum bei 40°, wonach man 19,2 g 5-Carbamoyl-10-nitro-5H-dibenz[b,f]azepin als $BF_3$-Addukt erhält. Zur Freisetzung des Endproduktes wird das $BF_3$-Addukt in 150 ml Wasser angeschlämmt, 15 Std. gerührt, abgesaugt und mit Wasser neutral gewaschen. Nach Trocknen erhält man 5-Carbamoyl-10-nitro-5H-dibenz[b,f]azepin, welches mit dem gemäss Beispiel 21 erhaltenen Produkt identisch ist.

Ausbeute: 13,5 g, 95,7% d. Th.

Beispiel 24

Zu einer Suspension von 26,3 g (0,1 Mol) 5-Cyano-10-nitro-5H-dibenz[b,f]azepin in 40 ml Essigsäure lässt man 50 ml einer Lösung von 15 Gew.-% $BF_3$ in Essigsäure zufliessen und rührt, bis unter Selbsterwärmung auf 40° vollständige Auflösung erfolgt ist. Man tropft dann 10 ml Wasser im Laufe von 10 Min. zu, wobei sich die Lösung auf 50° erwärmt, hält 10 Min. auf dieser Temperatur und tropft anschliessend weitere 300 ml Wasser langsam zu. Die entstandene Kristallsuspension wird 1 Std. bei Raumtemperatur gerührt, abgesaugt und mit Wasser neutral gewaschen. Nach Trocknen bei 60° im Vakuum erhält man 5-Carbamoyl-10-nitro-5H-dibenz[b,f]azepin, welches mit dem gemäss Beispiel 21 erhaltenen Produkt identisch ist.

Ausbeute 27,4 g, 97,5% d. Th.

Beispiel 25

10,0 g (0,035 Mol) 5-Carbamoyl-10-nitro-5H-dibenz[b,f]azepin werden in der Mischung von 100 ml Essigsäure und 50 ml konz. Salzsäure gelöst und nach Zusatz von 1,0 g Palladiumkohle (5%) bei Raumtemperatur und Normaldruck hydriert. Nach Aufnahme von 1,700 ml (109% d. Th.) Wasserstoff wird die Hydrierung unterbrochen, der Katalysator abfiltriert und das Filtrat im Vakuum auf ca. ¼ des Volumens eingeengt. Nach Zusatz von 400 ml Wasser lässt man einige Stunden bei 5° kristallisieren, saugt ab und wäscht mit Wasser neutral, wonach man das

5-Carbamoyl-10-oxo-10,11-dihydro-5H-dibenz-[b,f]azepin,

erhält, welches aufgrund des IR-Spektrums mit authentischem Material identisch ist.

Ausbeute: 7,0 g, 78,6% d. Th.

Ein ähnliches Resultat wird unter Verwendung von Platinkohle (5%) als Katalysator erhalten.

Beispiel 26

7,0 g (0,025 Mol) 5-Carbamoyl-10-nitro-5H-dibenz[b,f]azepin werden in der Mischung von 100 ml Essigsäure und 50 ml konz. Salzsäure gelöst. Unter Rühren setzt man bei einer Temperatur von 30–40° 12 g Eisenpulver im Laufe von 15 Min. zu, rührt noch eine Stunde bei 40°, filtriert warm vom Ungelösten ab und wäscht dreimal mit Essigsäure nach. Das Filtrat wird im Vakuum vollständig eingedampft, der Rückstand mit 100 ml Wasser aufgenommen und mehrere Stunden gerührt. Man filtriert ab, wäscht mit Wasser neutral und trocknet im Vakuum bei 60°. Man erhält 5-Carbamoyl-10-oxo-10,11-dihydro-5H-dibenz[b,f]azepin, welches aufgrund des IR-Spektrums mit authentischem Material ist.

Ausbeute: 5,5 g, 88,6% d. Th.

Beispiel 27

7,9 g (0,03 Mol) 5-Cyano-10-nitro-5H-dibenz[b,f]azepin werden in 150 ml Äthanol suspendiert und mit 50 ml konz. Salzsäure versetzt. Bei einer Temperatur von 40° fügt man im Laufe von 15 Min. 15 g Eisenpulver unter lebhaftem Rühren zu, wobei die Temperatur auf 55° ansteigt. Man lässt eine Stunde bei 55° nachrühren, filtriert warm vom ungelösten Material ab und wäscht dreimal mit je 25 ml Äthanol nach. Das Filtrat wird auf ein Volumen von 80 ml eingedampft und unter Rühren langsam mit 400 ml Eiswasser versetzt. Man saugt die grauweisse Fällung ab, wäscht mit Wasser neutral und trocknet im Vakuum bei 80°. Man erhält

5-Cyano-10-oxo-10,11-dihydro-5H-dibenz-[b,f]azepin,

welches nach dem Umlösen aus Äthanol bei 154–156° schmilzt.

Ausbeute: 4,8 g, 68,4% d. Th.

Die analytischen und spektroskopischen Daten sind im Einklang mit der angenommenen Struktur.

Beispiel 28

52,6 g (0,2 Mol) 5-Cyano-10-nitro-5H-dibenz[b,f]azepin werden in der Mischung von 400 ml Toluol und 200 ml Äthanol suspendiert. Beim Zusatz von 130 ml Salzsäure (konz.) geht das Material unter schwacher Erwärmung in Lösung. Man heizt auf 40° auf und setzt dann im Laufe von 10 Min. eine Lösung von 113 g (0,5 Mol) $SnCl_2 \cdot 2\,H_2O$ in 90 ml konz. Salzsäure zu. Die Temperatur steigt auf 55° und wird anschliessend noch 20 Min. auf 55° gehalten. Man lässt abkühlen, trennt die organische Phase ab und extrahiert die wässrige Phase noch mehrmals mit Toluol. Die vereinigten Toluolextrakte werden mit Wasser neutral gewaschen, über Natriumsulfat getrocknet und im Vakuum eingedampft. Das zurückbleibende Rohprodukt wird mit 100 ml Isopropanol angeschlämmt, abgesaugt und mit wenig Isopropanol kalt gewaschen. Man erhält 5-Cyano-10-oxo-10,11-dihydro-5H-dibenz[b,f]azepin, welches mit dem gemäss Beispiel 27 erhaltenen Produkt identisch ist.

Ausbeute: 29,0 g, 62% d. Th.

Beispiel 29

5,0 g (0,02 Mol) 5-Cyano-10-nitro-5H-dibenz-[b,f]azepin werden bei 60° in der Mischung von 80 ml Äthanol und 80 ml Essigsäure gelöst. Man gibt unter Rühren im Laufe von 10 Min. 10 g Zinkstaub portionenweise zu, wobei die Temperatur auf 80° steigt. Nach dem Abklingen der Reaktion werden bei Raumtemperatur 40 ml konz. Salzsäure zugesetzt. Man rührt das Reaktionsgemisch während 5 Stunden, filtriert vom Ungelösten ab, dampft das Filtrat zur Trockne ein und nimmt den Rückstand mit 50 ml Wasser auf. Nach mehrmaligem Umkristallisieren des ausgefallenen Rohprodukts erhält man reines

5-Cyano-10-oxo-10,11-dihydro-5H-dibenz-[b,f]azepin,

welches mit dem gemäss Beispiel 27 erhaltenen Produkt identisch ist.

Ausbeute: 2,3 g, 50% d. Th.

Beispiel 30

26,3 g (0,1 Mol) 5-Cyano-10-nitro-5H-dibenz-[b,f]azepin werden in einer Mischung von 150 ml Essigsäure und 100 ml konz. Salzsäure suspendiert. Bei 30° gibt man unter Rühren portionenweise 40 g Eisenpulver im Laufe von 30 Min. zu und hält durch Kühlung die Temperatur bei 60°. Man rührt weitere 30 Min. bei 50–60°, filtriert vom Ungelösten ab und wäscht mit Essigsäure nach. Das Filtrat wird mit dem doppelten Volumen Wasser versetzt und dreimal mit je 100 ml Methylenchlorid extrahiert. Die vereinigten organischen Phasen werden mit Wasser gewaschen, über Natriumsulfat getrocknet und eingedampft. Der kristalline Rückstand wird in 100 ml Isopropanol angeschlämmt und abgesaugt. Das so erhaltene

5-Cyano-10-oxo-10,11-dihydro-5H-dibenz[b,f]azepin

ist mit dem nach Beispiel 27 erhaltenen Produkt identisch.

Ausbeute: 21,8 g, 93,2% d. Th.

Beispiel 31

5,2 g (0,02 Mol) 5-Cyano-10-nitro-5H-dibenz-[b,f]azepin werden in der Mischung von 100 ml Chlorbenzol und 50 ml Äthanol suspendiert. Beim anschliessenden Zusatz von 50 ml konz. Salzsäure erwärmt sich die Mischung auf 35°. Man fügt bei dieser Temperatur unter lebhaftem Rühren 20 g Eisenpulver im Laufe von 5 Min. portionenweise zu, wobei sich das Reaktionsgemisch in wenigen Minuten auf 60° erwärmt. Man lässt anschliessend 3 Std. nachrühren und während dieser Zeit auf 25° abkühlen, filtriert vom Eisenschlamm ab und wäscht mit Äthanol und Wasser mehrmals nach. Die organische Phase des Filtrats liefert nach Auswaschen mit Wasser, Trocknen und Eindampfen ein kristallines Rohprodukt, das aus Isopropanol umkristallisiert wird. Das so erhaltene

5-Cyano-10-oxo-10,11-dihydro-5H-dibenz-[b,f]azepin,

ist mit dem gemäss Beispiel 27 erhaltenen Produkt identisch.

Ausbeute: 3,8 g 81% d. Th.

Ein mit Toluol statt Chlorbenzol als Lösungsmittel analog durchgeführter Reaktionsansatz lieferte 84% Ausbeute des genannten Endprodukts.

Beispiel 32

26,3 g (0,1 Mol) 5-Cyano-10-nitro-5H-dibenz-[b,f]azepin werden zusammen mit 40 g Eisenpulver in der Mischung von 250 ml Toluol und 125 ml Äthanol suspendiert. Man lässt 100 ml konz. Salzsäure unter intensivem Rühren im Laufe von 75 Min. zutropfen, wobei die Temperatur bis auf 60° ansteigt. Nach 10-stündigem Rühren bei Raumtemperatur wird das Reaktionsgemisch analog Beispiel 31 aufgearbeitet, wonach man das 5-Cyano-10-oxo-10,11-dihydro-5H-dibenz[b,f]azepin erhält, welches mit dem gemäss Beispiel 27 erhaltenen Produkt identisch ist.

Ausbeute: 19,8 g, 84,6% d. Th.

Beispiel 33

Man löst unter Eiskühlung 2,0 g (0,0085 Mol) 5-Cyano-10-oxo-10,11-dihydro-5H-dibenz[b,f]azepin in 10 ml konz. Schwefelsäure, lässt 30 Min. bei 0–5° stehen und tropft die Lösung dann in 200 ml Eiswasser ein. Die flockige Fällung wird abgesaugt, mit Wasser neutral gewaschen, getrocknet und aus Isopropanol umkristallisiert. Man erhält

5-Carbamoyl-10-oxo-10,11-dihydro-5H-dibenz-[b,f]azepin,

welches aufgrund des IR-Spektrums mit authentischem Material identisch ist.

Ausbeute: 1,4 g, 65% d. Th.

Beispiel 34

Ein Gemisch von 1,0 g (0,0043 Mol) 5-Cyano-10-oxo-10,11-dihydro-5H-dibenz[b,f]azepin, 8 ml Essigsäure und 2 ml konz. Schwefelsäure wird 48 Std. bis zur vollständigen Auflösung gerührt. Die erhaltene Lösung tropft man unter Rühren in 100 ml Eiswasser, filtriert die flockige Fällung ab, wäscht mit Wasser neutral und trocknet bei 50° im Vakuum, wonach man 5-Carbamoyl-10-oxo-10,11-dihydro-5H-dibenz[b,f]azepin erhält, welches aufgrund des IR-Spektrums mit authentischem Material identisch ist.

Ausbeute: 0,9 g, 84% d. Th.

Beispiel 35

Die Lösung von 2,0 g (0,0085 Mol) 5-Cyano-10-oxo-10,11-dihydro-5H-dibenz[b,f]azepin in 20 ml 98%iger Ameisensäure wird 8 Std. in einem Bad von 110–120° erhitzt. Danach trägt man die Lösung in 100 ml Eiswasser ein, filtriert, wäscht mit Wasser neutral und trocknet bei 50° im Vakuum. Das erhaltene 5-Carbamoyl-10-oxo-10,11-dihydro-5H-dibenz[b,f]azepin ist aufgrund des IR-Spektrums identisch mit authentischem Material.

Ausbeute: 1,8 g, 84% d. Th.

Beispiel 36

1,0 g (0,0043 Mol) 5-Cyano-10(11)-oxo-10,11-dihydro-5H-dibenz[b,f]azepin wird mit 20 g Polyphosphorsäure verrieben und bis zur vollständigen Auflösung mehrere Tage bei Raumtemperatur stehen gelassen. Man versetzt anschliessend mit überschüssigem Wasser in kleinen Portionen, saugt die gelblichweisse Fällung ab und wäscht mit Wasser neutral. Nach Umkristallisieren aus Chlorbenzol erhält man

5-Carbamoyl-10-oxo-10,11-dihydro-5H-dibenz-[b,f]azepin,

welches aufgrund des IR-Spektrums identisch mit authentischem Material ist.

Ausbeute: 0,75 g, 70% d. Th.

Beispiel 37

Zu einer Suspension von 1,0 g (0,0043 Mol)

5-Cyano-10-oxo-10,11-dihydro-5H-dibenz-[b,f]azepin

in 20 ml Methanol lässt man unter Rühren bei Raumtemperatur 5,0 ml $H_2O_2$ (30%ig) zulaufen und gibt nach 30 Min. 5,0 g Natriumhydrogencarbonat zu. Nach dreistündigem Rühren wird das Ungelöste abfiltriert und einmal mit Methanol sowie mehrmals mit Wasser gewaschen, wonach man

5-Carbamoyl-10-oxo-10,11-dihydro-5H-dibenz-[b,f]azepin

erhält, welches aufgrund des IR-Spektrums identisch ist mit authentischem Material.

Ausbeute: 0,6 g, 55% d. Th.

Beispiel 38

In eine Suspension von 3,0 g (0,013 Mol)

5-Cyano-10-oxo-10,11-dihydro-5H-dibenz-[b,f]azepin

in der Mischung von 30 ml Essigsäure und 3 ml Wasser leitet man unter Rühren $BF_3$-Gas so lange ein, bis die zunächst stark exotherme Reaktion abklingt und hält durch Aussenkühlung die Temperatur auf 50°. Man lässt abkühlen und tropft zur klaren Reaktionslösung unter Eiskühlung 100 ml Wasser, filtriert die farblose Fällung ab, wäscht den Filterrückstand mit Wasser neutral und kristallisiert aus Acetonitril um. Das erhaltene

5-Carbamoyl-10-oxo-10,11-dihydro-5H-dibenz-[b,f]azepin,

ist aufgrund des IR-Spektrums mit authentischem Material identisch.

Ausbeute: 2,3 g, 71% d. Th.

Beispiel 39

2,0 g (0,0085 Mol) 5-Cyano-10-oxo-10,11-dihydro-5H-dibenz[b,f]azepin werden portionenweise unter Rühren in 40 ml einer Lösung von 10 Gew.-% $BF_3$ in Essigsäure eingetragen, wonach man im Laufe von 15 Min. bei 20° eine klare Lösung erhält. Nach dreistündigem Stehen werden unter Eiskühlung 100 ml Wasser zugesetzt und die Lösung durch Zugabe von Natriumhydroxidlösung auf pH 6 eingestellt. Das ausgefallene rohe 5-Carbamoyl-10-oxo-10,11-dihydro-5H-dibenz[b,f]azepin wird abgesaugt, mit Wasser gewaschen und getrocknet. Aufgrund des IR-Spektrums ist es identisch mit authentischem Material.

Ausbeute: 1,6 g, 74,8% d. Th.

Beispiel 40

Zu einer Suspension von 11,7 g (0,05 Mol)

5-Cyano-10-oxo-10,11-dihydro-5H-dibenz-[b,f]azepin

in 60 ml Essigsäure fügt man 10,3 g (0,055 Mol) des Komplexes $BF_3 \cdot 2CH_3COOH$ zu und lässt die Temperatur ohne Aussenkühlung auf 35° ansteigen, wobei sich das Ausgangsmaterial langsam auflöst. Nach ca. 1 Std. beginnt ein $BF_3$-Addukt auszukristallisieren. Man rührt 4 Std. bei Raumtemperatur, filtriert ab und wäscht mit Essigsäure nach. Das Zwischenprodukt wird in 100 ml Wasser angeschlämmt und nach Abstumpfen mit Natriumacetat auf pH 6 eine Stunde gerührt. Nach Absaugen und Waschen mit Wasser erhält man 9,0 g 5-Carbamoyl-10-oxo-10,11-dihydro-5H-dibenz-[b,f]azepin, welches aufgrund des IR-Spektrums mit authentischem Material identisch ist. Aus dem Filtrat des Zwischenprodukts lassen sich nach Eindampfen durch fraktionierte Kristallisation aus Methanol/Wasser weitere 1,2 g des Produkts erhalten.

Totalausbeute: 10,2 g, 81% d. Th.

Beispiel 41

Zu einer Suspension von 23,4 g (0,1 Mol)

5-Cyano-10-oxo-10,11-dihydro-5H-dibenz-[b,f]azepin

in 40 ml Essigsäure fügt man 50 ml einer Lösung von 15 Gew.-% $BF_3$ in Essigsäure zu und rührt, bis die schwache Wärmeentwicklung abklingt. Tropfenweises Zufügen von 15 ml Wasser führt unter Temperaturanstieg auf 40–45° zu einer klaren tiefblauen Lösung. Man hält 15 Min. auf 40° und setzt dann weitere 135 ml Wasser langsam zu. Die erhaltene kristalline Ausfällung wird mehrere Stunden bei Raumtemperatur gerührt, dann abgesaugt und mit Wasser neutral gewaschen. Nach Trocknen bei 50° im Vakuum erhält man

5-Carbamoyl-10-oxo-10,11-dihydro-5H-dibenz[b,f]azepin,

welches aufgrund des IR-Spektrums identisch mit authentischem Material ist.

Ausbeute: 24,2 g, 96,0% d. Th.

Beispiel 42

In eine Suspension von 23,4 g (0,1 Mol)

5-Cyano-10-oxo-10,11-dihydro-5H-dibenz-[b,f]azepin

in 230 ml Chlorbenzol lässt man bei Raumtemperatur unter Rühren die Mischung von 15,4 ml (0,11 Mol) des Komplexes $BF_3 \cdot 2\,CH_3COOH$ mit 38 ml Essigsäure rasch einlaufen. Es entsteht unter leichter Erwärmung eine klare bräunliche Lösung, aus der nach ca. 10 Min. ein kristallines $BF_3$-Addukt ausfällt. Man hält 30 Min. auf 5°, filtriert ab und wäscht mit Chlorbenzol nach. Das trockene Zwischenprodukt wird in 200 ml Wasser angeschlämmt, 30 Min. gerührt, abgesaugt und mit Wasser neutral gewaschen. Nach Trocknen im Vakuum bei 60° erhält man 5-Carbamoyl-10-oxo-10,11-dihydro-5H-dibenz[b,f]azepin, welches aufgrund des IR-Spektrums identisch mit authentischem Material ist.

Ausbeute: 24,1 g, 95,6% d. Th.

Beispiel 43

1,0 g (0,004 Mol) 5-Cyano-10-nitro-5H-dibenz-[b,f]azepin wird bei 60° in der Mischung von 40 ml Äthanol und 20 ml Essigsäure gelöst und unter lebhaftem Rühren im Laufe von 10 Min. mit 2,0 g Zinkstaub versetzt. Man lässt 15 Min. nachrühren, filtriert warm vom Ungelösten ab und wäscht mit Äthanol und Wasser nach. Das Filtrat wird zur Trockne eingedampft und mit 50 ml Wasser aufgenommen. Nach Absaugen, Waschen mit Wasser und Trocknen erhält man rohes

5-Cyano-10-isonitroso-10,11-dihydro-5H-dibenz-[b,f]azepin,

welches nach Umkristallisieren aus Äthanol bei 185° unter Zersetzung schmilzt. Die analytischen und spektroskopischen Daten sind mit der angenommenen Struktur im Einklang.

Ausbeute: 0,9 g, 95% d. Th.

Beispiel 44

50 g (0,19 Mol) 5-Cyano-10-nitro-5H-dibenz-[b,f]azepin werden in 500 ml Pyridin gelöst und nach Zusatz von 10 g Palladiumkohle (5%) bei Raumtemperatur und Normaldruck hydriert. Nach 2 Std. sind 7,960 ml (93% d. Th.) Wasserstoff aufgenommen, und die Hydrierung kommt zum Stillstand. Man filtriert vom Katalysator ab, dampft das Lösungsmittel im Vakuum ab und kristallisiert das zurückbleibende Rohprodukt aus Methanol/Wasser um, wonach man das

5-Cyano-10-isonitroso-10,11-dihydro-5H-dibenz-[b,f]azepin

erhält, welches mit dem gemäss Beispiel 43 erhaltenen Produkt identisch ist.

Ausbeute: 35,6 g, 75,2% d. Th.

Beispiel 45

2,5 g (0,01 Mol) 5-Cyano-10-isonitroso-10,11-dihydro-5H-dibenz[b,f]azepin werden in der Mischung von 25 ml Toluol, 15 ml Äthanol und 10 ml Salzsäure (konz.) suspendiert. Man rührt die Suspension 30 Min. bei 50°, trennt die Toluolphase ab und dampft sie im Vakuum zur Trockne ein. Der kristalline Rückstand wird in 10 ml Isopropanol angeschlämmt, abgesaugt und zweimal mit Isopropanol gewaschen, wonach man

5-Cyano-10-oxo-10,11-dihydro-5H-dibenz[b,f]-azepin

erhält, welches mit dem gemäss Beispiel 27 erhaltenen Produkt identisch ist.

Ausbeute: 0,8 g, 35% d. Th.

Beispiel 46

26,3 g (Mol) 5-Cyano-10-nitro-5H-dibenz[b,f] azepin werden in der Mischung von 265 ml Äthylenglycol-monoäthyläther und 75 ml konzentrierter Salzsäure suspendiert. Man setzt bei 40° im Laufe von 40 Minuten 40 g Eisenpulver in kleinen Portionen zu und hält durch Aussenkühlung die Temperatur bei 40°. Man rührt weitere 2 Stunden bei Raumtemperatur, heizt dann auf 80° auf, wobei das ausgefallene Produkt sich wieder löst, und filtriert durch eine geheizte Nutsche, die mit Äthylenglycol-monoäthyläther nachgewaschen wird. Die im Filtrat einsetzende Kristallisation wird durch Zugabe von 200 ml Wasser vervollständigt. Nach einstündigem Kühlen im Eisbad wird filtriert und mit einer Mischung von Äthylenglycol-monoäthyläther und Wasser im Verhältnis 1 : 1 nachgewaschen. Man erhält

5-Cyano-10-oxo-10,11-dihydro-5H-dibenz[b,f]-azepin,

das durch Vergleich der IR-Spektren mit Material aus Beispiel 27 identifiziert wird.

Ausbeute: 19,0 g, 81,2% d. Th.

**Patentansprüche für die Vertragsstaaten BE, CH, DE, FR, GB, IT, LI, LU, NL, SE**

1. Verfahren zur Herstellung von 5-Carbamoyl-10-oxo-10,11-dihydro-5H-dibenz[b,f]azepin der Formel

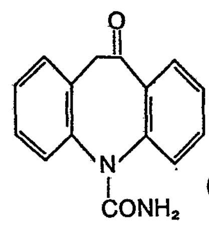

(III) ,

dadurch gekennzeichnet, dass man 5-Cyano-5H-dibenz[b,f]azepin der Formel

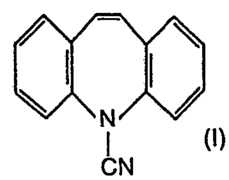

(I)

in einem unter den Bedingungen der Nitrierung stabilen Lösungsmittel in einem Temperaturbereich von ca. 0–120 °C, insbesondere von 40–80 °C, nitriert und das erhaltene 5-Cyano-10-nitro-5H-dibenz[b,f]azepin der Formel

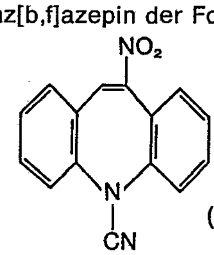

(II)

entweder (a) mittels die 10-Nitrogruppe nicht beeinträchtigender Hydrolysemethoden zum 5-Carbamoyl-10-nitro-5H-dibenz[b,f]azepin der Formel

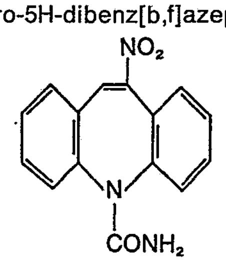

(IV),

hydrolysiert, hierin die 10-Nitrogruppe zur 10-Isonitrosogruppe reduziert und die erhaltene 10-Isonitrosoverbindung zur Verbindung der Formel III hydrolysiert, oder

(b) in einem Temperaturbereich von 10–100 °C, vorzugsweise von 30–70 °C, zum 5-Cyano-10-isonitroso-10,11-dihydro-5H-dibenz[b,f]azepin der Formel

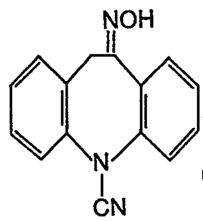

(VI)

reduziert und dieses entweder direkt oder über das 5-Cyano-10-oxo-10,11-dihydro-5H-dibenz[b,f]-azepin der Formel

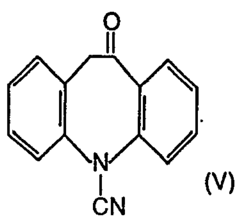

(V)

zur Verbindung der Formel III hydrolysiert, wobei die Hydrolyse der 5-Cyanogruppe in einem Temperaturbereich von −5–+150 °C, vorzugsweise 0–40 °C erfolgt.

2. Verfahren nach Anspruch 1, dadurch gekennzeichnet, dass man in der Verfahrensvariante (a) die Verbindung der Formel II hydrolysiert und die im Reaktionsgemisch enthaltene Verbindung der Formel IV, ohne diese zu isolieren, reduziert, und das im Reaktionsgemisch vorliegende Reduktionsprodukt, ohne dieses zu isolieren, zur Verbindung der Formel III hydrolysiert.

3. Verfahren nach Anspruch 1, dadurch gekennzeichnet, dass man in der Verfahrensvariante (b) die Verbindung der Formel II reduziert und das Reaktionsprodukt, ohne dieses in reiner Form zu isolieren, zur Verbindung der Formel V hydrolysiert.

4. Verfahren nach Anspruch 1, dadurch gekennzeichnet, dass man in der Verfahrensvariante (b) bei der Reduktion der Verbindung der Formel II mittels eines Metalls zur Verbindung der Formel IV ein bei der Reduktion entstehende Metallsalze in Lösung haltendes Lösungsmittel verwendet.

5. Verfahren nach Anspruch 1, dadurch gekennzeichnet, dass man bei der Verfahrensvariante (b) in der Verbindung der Formel VI sowohl die 5-Cyanogruppe als auch die 10-Isonitrosogruppe im gleichen Reaktionsansatz hydrolysiert, um zur Verbindung der Formel III zu gelangen.

6. 5-Cyano-10-nitro-5H-dibenz[b,f]azepin.

7. 5-Carbamoyl-10-nitro-5H-dibenz[b,f]azepin.

8. 5-Cyano-10-oxo-10,11-dihydro-5H-dibenz-[b,f]azepin.

9. 5-Cyano-10-isonitroso-10,11-dihydro-5H-dibenz[b,f]azepin.

10. $BF_3$-Addukt des 5-Carbamoyl-10-nitro-5H-dibenz[b,f]azepins.

**Patentansprüche für den Vertragsstaat AT**

1. Verfahren zur Herstellung von 5-Carbamoyl-10-oxo-10,11-dihydro-5H-dibenz[b,f]azepin der Formel

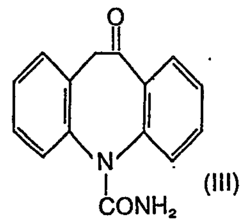

(III),

dadurch gekennzeichnet, dass man 5-Cyano-5H-dibenz[b,f]azepin der Formel

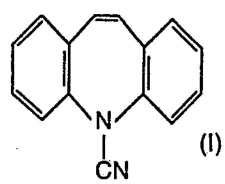

(I)

in einem unter den Bedingungen der Nitrierung stabilen Lösungsmittel in einem Temperaturbereich von ca. 0–120 °C, insbesondere von 40–80 °C, nitriert, und das erhaltene 5-Cyano-10-nitro-5H-dibenz[b,f]azepin der Formel

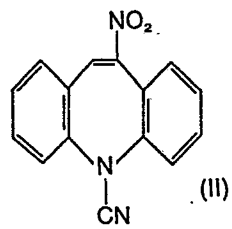

(II)

(a) mittels die 10-Nitrogruppe nicht beeinträchtigender Hydrolysemethoden zum 5-Carbamoyl-10-nitro-5H-dibenz[b,f]azepin der Formel

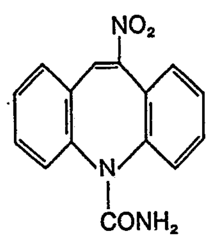

(IV)

hydrolysiert, hierin die 10-Nitrogruppe zur 10-Isonitrosogruppe reduziert und die erhaltene 10-Isonitrosoverbindung zur Verbindung der Formel III hydrolysiert, oder

(b) in einem Temperaturbereich von 10–100 °C, vorzugsweise von 30–70 °C, zum 5-Cyano-10-isonitroso-10,11-dihydro-5H-dibenz[b,f]azepin der Formel

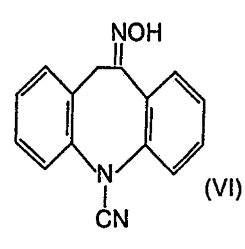

(VI)

reduziert, und dieses entweder direkt oder über das 5-Cyano-10-oxo-10,11-dihydro-5H-dibenz[b,f]-azepin der Formel

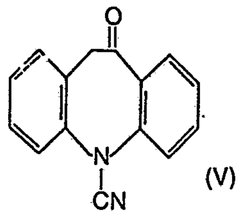
(V)

zur Verbindung der Formel III hydrolysiert, wobei die Hydrolyse der 5-Cyanogruppe in einem Temperaturbereich von −5–+150 °C, vorzugsweise von 0–40 °C, erfolgt.

2. Verfahren nach Anspruch 1, dadurch gekennzeichnet, dass man in der Verfahrensvariante (a) die Verbindung der Formel II hydrolysiert und die im Reaktionsgemisch enthaltene Verbindung der Formel IV, ohne diese zu isolieren, reduziert, und das im Reaktionsgemisch vorliegende Reduktionsprodukt, ohne dieses zu isolieren, zur Verbindung der Formel III hydrolysiert.

3. Verfahren nach Anspruch 1, dadurch gekennzeichnet, dass man in der Verfahrensvariante (b) die Verbindung der Formel II reduziert und das Reaktionsprodukt, ohne dieses in reiner Form zu isolieren, zur Verbindung der Formel V hydrolysiert.

4. Verfahren nach Anspruch 1, dadurch gekennzeichnet, dass man in der Verfahrensvariante (b) bei der Reduktion der Verbindung der Formel II mittels eines Metalls zur Verbindung der Formel VI ein bei der Reduktion entstehende Metallsalze in Lösung haltendes Lösungsmittel verwendet.

5. Verfahren nach Anspruch 1, dadurch gekennzeichnet, dass man bei der Verfahrensvariante (b) in der Verbindung der Formel VI sowohl die 5-Cyanogruppe als auch die 10-Isonitrosogruppe im gleichen Reaktionsansatz hydrolysiert, um zur Verbindung der Formel III zu gelangen.

6. Verfahren zur Herstellung von 5-Cyano-10-nitro-5H-dibenz[b,f]azepin der Formel (II) nach Anspruch 1, dadurch gekennzeichnet, dass man 5-Cyano-5H-dibenz[b,f]azepin der Formel (I) in einem unter den Bedingungen der Nitrierung stabilen Lösungsmittel in einem Temperaturbereich von ca. 0–120 °C, insbesondere von 40–80 °C, nitriert.

7. Verfahren zur Herstellung von 5-Carbamoyl-10-nitro-5H-dibenz[b,f]azepin der Formel (IV) nach Anspruch 1, dadurch gekennzeichnet, dass man 5-Cyano-10-nitro-5H-dibenz[b,f]azepin der Formel (II) mittels die an der Doppelbindung stehende 10-Nitrogruppe nicht beeinträchtigender Hydrolysemethoden hydrolysiert.

8. Verfahren zur Herstellung von 5-Cyano-10-oxo-10,11-dihydro-5H-dibenz[b,f]azepin der Formel (V) nach Anspruch 1, dadurch gekennzeichnet, dass man 5-Cyano-10-nitro-5H-dibenz[b,f]-azepin der Formel (II) in einem Temperaturbereich von 10–100 °C, vorzugsweise von 30–70 °C, zum 5-Cyano-10-isonitroso-10,11-dihydro-5H-dibenz-[b,f]azepin der Formel (VI) reduziert und dieses zum 5-Cyano-10-oxo-10,11-dihydro-5H-dibenz[b,f]azepin hydrolysiert.

9. Verfahren zur Herstellung von 5-Cyano-10-isonitroso-10,11-dihydro-5H-dibenz[b,f]azepin der Formel (VI), nach Anspruch 1, dadurch gekennzeichnet, dass man 5-Cyano-10-nitro-5H-dibenz[b,f]azepin der Formel (II) in einem Temperaturbereich von 10–100 °C, vorzugsweise von 30–70 °C, reduziert.

10. Verfahren zur Herstellung des $BF_3$-Addukts des 5-Carbamoyl-10-nitro-5H-dibenz[b,f]azepins, dadurch gekennzeichnet, dass man das nach Anspruch 1 erhaltene 5-Cyano-10-nitro-5H-dibenz-[b,f]azepin der Formel (II), mittels einer Lösung von $BF_3$ in Essigsäure hydrolysiert.

**Claims for the Contracting States: BE, CH, DE, FR, GB, IT, LI, LU, NL, SE**

1. A process for the preparation of 5-carbamoyl-10-oxo-10,11-dihydro-5H-dibenz[b,f]azepine of the formula

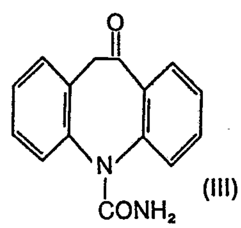
(III)

which process comprises nitrating 5-cyano-5H-dibenz[b,f]azepine of the formula

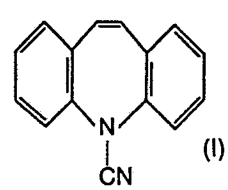
(I)

in a solvent which is stable under the nitration conditions, said nitration being effected in the temperature range from about 0° to 120 °C, preferably from 40° to 80 °C, and either
(a) by a method of hydrolysis which does not impair the 10-nitro group, hydrolysing the resultant 5-cyano-10-nitro-5H-dibenz[b,f]azepine of the formula

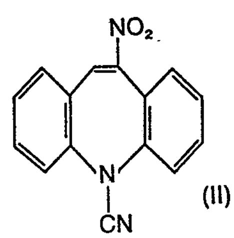
(II)

to give 5-carbamoyl-10-nitro-5H-dibenz[b,f]-azepine of the formula

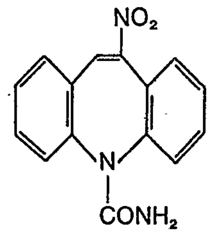

(IV)

reducing in said compound of formula IV the 10-nitro group to the 10-isonitroso group and hydrolysing the resultant 10-isonitroso compound to give a compound of the formula III, or

(b) in the temperature range from 10° to 100 °C, preferably from 30° to 70 °C, reducing the resultant 5-cyano-10-nitro-5H-dibenz[b,f]azepine of formula II to
5-cyano-10-isonitroso-10,11-dihydro-5H-dibenz[b,f]azepine
of the formula

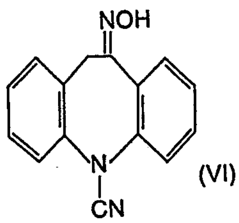

(VI)

and hydrolysing said compound of formula VI either direct or via
5-cyano-10-oxo-10,11-dihydro-5H-dibenz-[b,f]azepine
of the formula

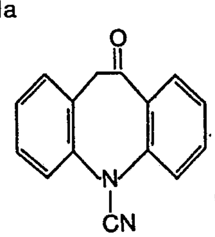

(V)

to give a compound of formula III, with the hydrolysis of the 5-cyano group being effected in the temperature range from −5° to +150 °C, preferably from 0° to 40 °C.

2. A process according to claim 1, which comprises in process variant (a) hydrolysing the compound of formula II, reducing the compound of formula IV contained in the reaction mixture, without isolating said compound, and hydrolysing the reduction product present in the reaction mixture, without isolating said product, to give a compound of formula III.

3. A process according to claim 1, which comprises in process variant (b) reducing the compound of formula II and hydrolysing the reaction product, without isolating said product in pure form, to give a compound of formula V.

4. A process according to claim 1, wherein the solvent employed in process variant (b) for the reduction of the compound of formula II with a metal to give a compound of formula VI is a solvent which keeps in solution metal salts which form during the reduction.

5. A process according to claim 1, which comprises in process variant (b) hydrolysing in the compound of formula VI both the 5-cyano group and the 10-isonitroso group in the same reaction mixture to give a compound of formula III.

6. 5-Cyano-10-nitro-5H-dibenz[b,f]azepine.

7. 5-Carbamoyl-10-nitro-5H-dibenz[b,f]azepine.

8. 5-Cyano-10-oxo-10,11-dihydro-5H-dibenz-[b,f]azepine.

9. 5-Cyano-10-isonitroso-10,11-dihydro-5H-dibenz[b,f]azepine.

10. The $BF_3$ adduct of 5-carbamoyl-10-nitro-5H-dibenz[b,f]azepine.

**Claims for the contracting State: AT**

1. A process for the preparation of
5-carbamoyl-10-oxo-10,11-dihydro-5H-dibenz[b,f]azepine
of the formula

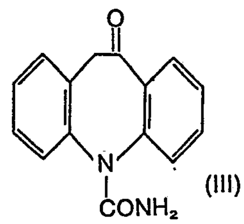

(III)

which process comprises nitrating 5-cyano-5H-dibenz[b,f]azepine of the formula

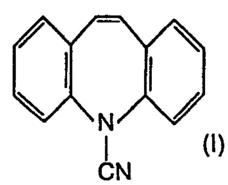

(I)

in a solvent which is stable under the nitration conditions, said nitration being effected in the temperature range from about 0° to 120 °C, preferably from 40° to 80 °C, and either

(a) by a method of hydrolysis which does not impair the 10-nitro group, hydrolysing the resultant 5-cyano-10-nitro-5H-dibenz[b,f] azepine of the formula

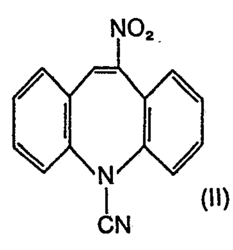

(II)

to give 5-carbamoyl-10-nitro-5H-dibenz[b,f]-azepine of the formula

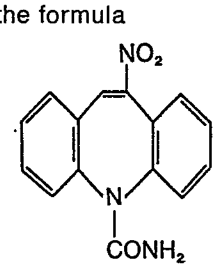

(IV)

reducing in said compound of formula IV the 10-nitro group to the 10-isonitroso group and hydrolysing the resultant 10-isonitroso compound to give a compound of the formula III, or

(b) in the temperature range from 10° to 100 °C, preferably from 30° to 70 °C, reducing the resultant 5-cyano-10-nitro-5H-dibenz[b,f]azepine of formula II to

5-cyano-10-isonitroso-10,11-dihydro-5H-dibenz[b,f]azepine

of the formula

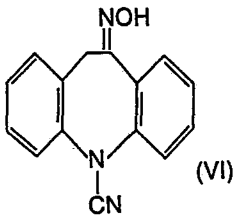
(VI)

and hydrolysing said compound of formula VI either direct or via

5-cyano-10-oxo-10,11-dihydro-5H-dibenz-[b,f]azepine

of the formula

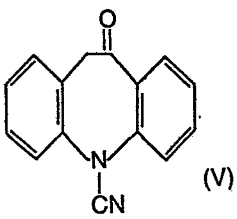
(V)

to give a compound of formula III, with the hydrolysis of the 5-cyano group being effected in the temperature range from −5° to +150 °C, preferably from 0° to 40 °C.

2. A process according to claim 1, which comprises in process variant (a) hydrolysing the compound of formula II, reducing the compound of formula IV contained in the reaction mixture, without isolating said compound, and hydrolysing the reduction product present in the reaction mixture, without isolating said product, to give a compound of formula III.

3. A process according to claim 1, which comprises in process variant (b) reducing the compound of formula II and hydrolysing the reaction product, without isolating said product in pure form, to give a compound of formula V.

4. A process according to claim 1, wherein the solvent employed in process variant (b) for the reduction of the compound of formula II with a metal to give a compound of formula VI is a solvent which keeps in solution metal salts which form during the reduction.

5. A process according to claim 1, which comprises in process variant (b) hydrolysing in the compound of formula VI both the 5-cyano group and the 10-isonitroso group in the same reaction mixture to give a compound of formula III.

6. A process for the preparation of 5-cyano-10-nitro-5H-dibenz[b,f]azepine of formula II according to claim 1, which comprises nitrating 5-cyano-5H-dibenz[b,f]azepine of formula I in a solvent which is stable under the nitration conditions, said nitration being effected in the temperature range from about 0° to 120 °C, preferably from 40° to 80 °C.

7. A process for the preparation of 5-carbamoyl-10-nitro-5H-dibenz[b,f]azepine of formula IV according to claim 1, which comprises hydrolysing 5-cyano-10-nitro-5H-dibenz[b,f]azepine of formula II by a method of hydrolysis which does not impair the 10-nitro group located at the double bond.

8. A process for the preparation of

5-cyano-10-oxo-10,11-dihydro-5H-dibenz-[b,f]azepine

of formula V according to claim 1, which comprises reducing 5-cyano-10-nitro-5H-dibenz-[b,f]azepine of formula II in the temperature range from 10° to 100 °C, preferably from 30° to 70 °C, to

5-cyano-10-isonitroso-10,11-dihydro-5H-dibenz[b,f]azepine

of formula VI and hydrolysing said compound of formula VI to give 5-cyano-10-oxo-10,11-dihydro-5H-dibenz[b,f]azepine.

9. A process for the preparation of

5-cyano-10-isonitroso-10,11-dihydro-5H-dibenz[b,f]azepine

of formula VI according to claim 1, which comprises reducing 5-cyano-10-nitro-5H-dibenz-[b,f]azepine of formula II in the temperature range from 10° to 100 °C, preferably from 30° to 70 °C.

10. A process for the preparation of the $BF_3$ adduct of 5-carbamoyl-10-nitro-5H-dibenz-[b,f]azepine, which comprises hydrolysing the 5-cyano-10-nitro-5H-dibenz[b,f]azepine of formula II obtained according to claim 1 with a solution of $BF_3$ in acetic acid.

**Revendications pour les Etats contractants: BE, CH, DE, FR, GB, IT, LI, LU, NL, SE**

1. Procédé de préparation de la

5-carbamoyl-10-oxo-10,11-dihydro-5H-dibenzo-[b,f]azépine

de formule III

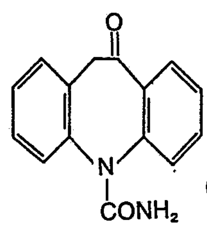
(III)

caractérisé en ce que l'on nitre la 5-cyano-5H-dibenzo[b,f]azépine de formule I

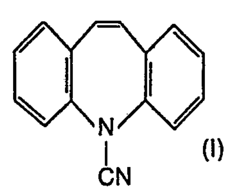
(I)

dans un solvant stable dans les conditions de la nitration, dans un intervalle de températures de 0 à 120 °C environ, plus spécialement de 40 à 40 °C, ce qui done la

5-cyano-10-nitro-5H-dibenzo[b,f]azépine

de formule II

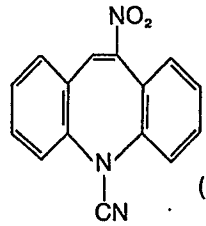
(II)

qu'on soument alors
(a) soit à l'hydrolyse par des techniques d'hydrolyse qui n'affectent pas le groupe 10-nitro,cette hydrolyse donnant la
5-carbamoyl-10-nitro-5H-dibenzo[b,f]azépine
de formule IV

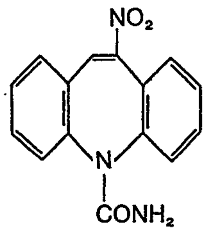
(IV)

après quoi on réduit le groupe 10-nitro en un groupe 10-isonitroso, et on hydrolyse le composé obtenu, isonitrosé en position 10, en le composé de formule III,
(b) soit à réduction dans l'intervalle de températures de 10 à 100 °C, de préférence de 30 à 70 °C, en la
5-cyano-10-isonitroso-10,11-dihydro-5H-dibenzo[b,f]azépine
de formule VI

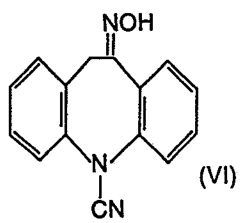
(VI)

qu'on hydrolyse, directement ou en passant par l'intermédiaire de la
5-cyano-10-oxo-10,11-dihydro-5H-dibenzo[b,f]azépine
de formule V

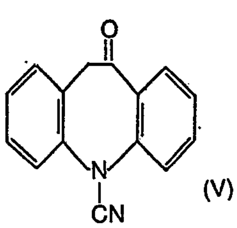
(V)

en le composé de formule III dans un intervalle de température de −5 à +150 °C de préférence de 0 à 40 °C.

2. Procédé selon la revendication 1, dans la variante opératoire a), caractérisé en ce qu'on hydrolyse le composé de formule II et on réduit le composé de formule IV contenu dans le mélange de réaction, sans l'isoler, puis on hydrolyse le produit de réduction présent dans le mélange de réaction sans l'isoler, en le composé de formule III.

3. Procédé selon la revendication 1, dans la variante opératoire b), caractérisé en ce que l'on réduit le composé de formule II et on hydrolyse le produit de réaction, sans l'isoler à l'état pur, en le composé de formule V.

4. Procédé selon la revendication 1, dans la variante opératoire b), caractérisé en ce que, à la réduction du composé de formule II en le composé de formule VI à l'aide d'un métal, on utilise un solvant contenant en solution des sels métalliques formés dans la réduction.

5. Procédé selon la revendication 1, dans la variante opératoire b), caractérisé en ce que, pour parvenir au composé de formule III, on hydrolyse à la fois le groupe 5-cyano et le groupe 10-isonitroso du composé de formule VI dans le même mélange de réaction.

6. La 5-cyano-10-nitro-5H-dibenzo[b,f]azépine.

7. La 5-carbamoyl-10-nitro-5H-dibenzo[b,f]azépine.

8. La 5-cyano-10-oxo-10,11-dihydro-5H-dibenzo[b,f]azépine.

9. La 5-cyano-10-isonitroso-10,11-dihydro-5H-dibenzo[b,f]azépine.

10. L'adduct de $BF_3$ de la 5-carbamoyl-10-nitro-5H-dibenzo[b,f]azépine.

**Revendications pour l'Etat contractant: AT**

1. Procédé de préparation de la
5-carbamoyl-10-oxo-10,11-dihydro-5H-dibenzo-[b,f]azépine
de formule III:

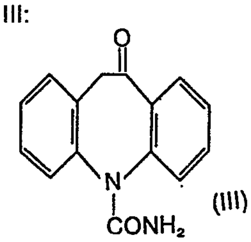
(III)

caractérisé en ce que l'on nitre la 5-cyano-5H-dibenzo(b,f]azépine de formule I

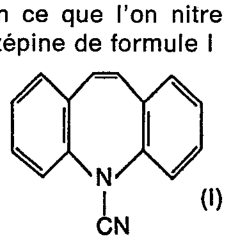
(I)

dans un solvant stable dans les conditions de la nitration, dans un intervalle de températures de 0 à 120 °C environ, plus spécialement de 40 à 80 °C, ce qui donne la 5-cyano-10-nitro-5H-dibenzo[b,f]-azépine de formule II

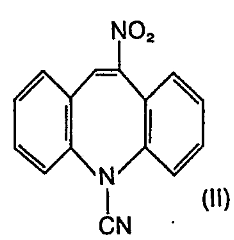
(II)

(a) que l'on hydrolyse par des modes opératoires d'hydrolyse qui n'affectent pas le groupe 10-nitro, en la 5-carbamoyl-10-nitro-5H-dibenzo[b,f]azépine de formule IV

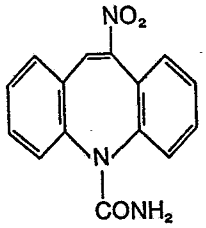
(IV)

dans laquelle on réduit le groupe 10-nitro en groupe 10-isonitroso, après quoi on hydrolyse le composé obtenu, portant un groupe isonitroso en position 10, en le composé de formule III, ou bien (b) qu'on réduit dans l'intervalle de températures de 10 à 100 °C, de préférence de 30 à 70 °C, en la 5-cyano-10-isonitroso-10,11-dihydro-5H-dibenzo[b,f]azépine de formule VI

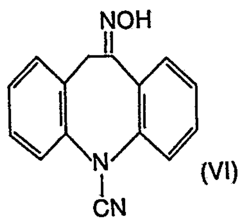
(VI)

qu'on hydrolyse directement ou en passant par l'intermédiaire de la 5-cyano-10-oxo-10,11-dihydro-5H-dibenzo[b,f]azépine de formule V

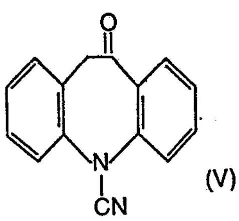
(V)

en le composé de formule III, l'hydrolyse du groupe 5-cyano étant effectuée dans un intervalle de températures allant de −5 à +150 °C, de préférence de 0 à 40 °C.

2. Procédé selon la revendication 1, caractérisé en ce que, dans la variante opératoire a), on hydrolyse le composé de formule II et on réduit le composé de formule IV contenu dans le mélange de réaction, sans l'isoler, puis on hydrolyse le produit de réduction présent dans le mélange de réaction, sans l'isoler, en le composé de formule III.

3. Procédé selon la revendication 1, caractérisé en ce que, dans la variante opératoire b), on réduit le composé de formule II et on hydrolyse le produit de réaction sans l'isoler à l'état pur, en le composé de formule V.

4. Procédé selon la revendication 1, caractérisé en ce que, dans la variante opératoire b), à la réduction du composé de formule II à l'aide d'un métal en le composé de formule VI, on utilise un solvant contenant en solution des sels métalliques formés dans la réduction.

5. Procédé selon la revendication 1, caractérisé en ce que, dans la variante opératoire b), on hydrolyse dans le composé de formule VI, à la fois le groupe 5-cyano et le groupe 10-isonitroso dans le même mélange de réaction pour parvenir au composé de formule III.

6. Procédé de preparation de la 5-cyano-10-nitro-5H-dibenzo[b,f]azépine de formule II selon la revendication 1, caractérisé en ce que l'on nitre la 5-cyano-5H-dibenzo[b,f]azépine de formule I dans un solvant stable dans les conditions de la nitration dans l'intervalle de températures de 0 à 120 °C environ, plus spécialement de 40 à 80 °C.

7. Procédé de préparation de la 5-carbamoyl-10-nitro-5H-dibenzo[b,f]azépine de formule IV selon la revendication 1, caractérisé en ce que l'on hydrolyse la 5-cyano-10-nitro-5H-dibenzo[b,f]azépine de formule II par des modes opératoires d'hydrolyse qui n'affectent pas le groupe 10-nitro fixé sur la double liaison.

8. Procédé de préparation de la
5-cyano-10-oxo-10,11-dihydro-5H-dibenzo-[b,f]azépine
de formule V selon la revendication 1, caractérisé en ce que l'on réduit la 5-cyano-10-nitro-5H-dibenzo[b,f]azépine de formule II dans l'intervalle de températures de 10 à 100 °C, de préférence de 30 à 70 °C, en la
5-cyano-10-isonitroso-10,11-dihydro-5H-dibenzo[b,f]azépine
de formule VI qu'on hydrolyse en la
5-cyano-10-oxo-10,11-dihydro-5H-dibenzo-[b,f]azépine.

9. Procédé de préparation de la
5-cyano-10-isonitroso-10,11-dihydro-5H-dibenzo[b,f]azépine
de formule VI, selon la revendication 1, caractérisé en ce que l'on réduit la 5-cyano-10-nitro-5H-dibenzo[b,f]azépine de formule II dans l'intervalle de températures de 10 à 100 °C, de préférence de 30 à 70 °C.

10. Procédé de préparation de l'adduct de la $BF_3$ de la
5-carbamoyl-10-nitro-5H-dibenzo[b,f]-azépine,
selon caractérisé en ce que l'on hydrolyse la
5-cyano-10-nitro-5H-dibenzo[b,f]-azépine
de formule III, obtenue selon la revendication 1, à l'aide d'une solution de $BF_3$ dans l'acide acétique.